(19) Europäisches Patentamt
European Patent Office
Office européen des brevets

(11) **EP 3 867 349 B1**

(12) **EUROPÄISCHE PATENTSCHRIFT**

(45) Veröffentlichungstag und Bekanntmachung des Hinweises auf die Patenterteilung:
**16.08.2023 Patentblatt 2023/33**

(21) Anmeldenummer: **20701702.1**

(22) Anmeldetag: **16.01.2020**

(51) Internationale Patentklassifikation (IPC):
***C12M 1/107*** *(2006.01)* ***C12M 1/34*** *(2006.01)*
***C12M 1/36*** *(2006.01)* ***C12P 5/02*** *(2006.01)*

(52) Gemeinsame Patentklassifikation (CPC):
**C12M 41/32; C12M 21/04; C12M 41/40; C12M 41/48; C12P 5/023;** Y02E 50/30

(86) Internationale Anmeldenummer:
**PCT/EP2020/050988**

(87) Internationale Veröffentlichungsnummer:
**WO 2020/152017 (30.07.2020 Gazette 2020/31)**

(54) **VERFAHREN ZUR AUTOMATISCHEN ÜBERWACHUNG VON BIOGASREAKTOREN**
METHOD FOR AUTOMATIC MONITORING OF BIOGAS REACTORS
PROCÉDÉ DE SURVEILLANCE AUTOMATIQUE DE RÉACTEURS DE BIOGAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.01.2019 DE 102019000581**

(43) Veröffentlichungstag der Anmeldung:
**25.08.2021 Patentblatt 2021/34**

(73) Patentinhaber: **Kazda, Marian**
**89075 Ulm (DE)**

(72) Erfinder: **Kazda, Marian**
**89075 Ulm (DE)**

(74) Vertreter: **Höfer, Friederike**
**Dreiköniggasse 10**
**89073 Ulm (DE)**

(56) Entgegenhaltungen:
**WO-A1-2014/128300 WO-A1-2014/140703**
**DE-A1-102011 110 638 DE-A1-102014 006 501**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 867 349 B1

## Beschreibung

### Gebiet der Erfindung

Die Erfindung bezieht sich auf ein Verfahren zur automatischen Überwachung von Biogasreaktoren.

### Hintergrund der Erfindung

Biogasanlagen werden zur Erzeugung von Biogas durch Vergärung von Biomasse eingesetzt. Als Biomasse kommen beispielweise Substrate aus dem landwirtschaftlichen Bereich, wie tierische Exkremente (Gülle), Energiepflanzen, wie Getreide, Zuckerrüben, Mais- und Grassilage, oder Material aus der Biotonne, Bioabfälle oder Abfallprodukte aus der Lebensmittelproduktion in Frage. Die Biomasse umfasst daher eine oder mehrere organische Substanzen.

In einer Biogasanlage, umfassend einen Bioreaktor, erfolgt der mikrobielle Abbau bzw. die Vergärung der organischen Substanzen in "anaerober" Art und Weise, d.h. ohne Sauerstoff. Die Abwesenheit von Sauerstoff ist erforderlich, weil die verwendeten methanbildenden Mikroorganismen Sauerstoff-empfindlich sind. Hauptprodukte des anaeroben Abbaus und damit des erzeugten Biogases sind Methan ($CH_4$) und Kohlendioxid ($CO_2$), die beide gasförmig sind und daher vom flüssigen und festen Gärsubstrat in den Gasraum des Biogasreaktors aufsteigen. Der als Nebenprodukt verbleibende Gärrest kann als Dünger Verwendung finden.

Das entstandene Gas aus der Biogasanlage wird häufig vor Ort in einem Blockheizkraftwerk (BHKW) zur Strom- und Wärmeerzeugung verwendet. Auch besteht die Möglichkeit, das gewonnene Gas zu Biomethan aufzureinigen und dieses ins Erdgasnetz einzuspeisen.

Die anaerobe Vergärung organischer Substanzen wird in Deutschland in ca. 9000 Biogasanlagen zur Methanerzeugung verwendet; in Europa gibt es ebenfalls mehrere Tausend Biogasanlagen. Die meisten Biogasreaktoren sind drucklose Behälter von teilweise mehr als 1000 $m^3$ Volumen, in denen unter Sauerstoffausschluss aus der zugeführten organischen Substanz, bevorzugt landwirtschaftliche Substrate oder organische Reststoffe, in mehreren Stufen Methan gebildet wird.

Der anaerobe Abbau- oder Vergärungsprozess der organischen Substanz in einem Bioreaktor zu Methan wird in 4 aufeinanderfolgende biochemische Stufen unterteilt. Bei zumeist kontinuierlich erfolgender Substratzufuhr zum Bioreaktor, finden diese 4 Stufen oder Phasen im Bioreaktor parallel statt. Die vier Stufen sind die Hydrolyse (1. Stufe), Acidogenese oder Versäuerungsstufe (2. Stufe), Acetogenese oder essigbildende Stufe (3. Stufe) und Methanogenese oder methanbildende Stufe (4. Stufe). Die 4 Stufen sind im Ablaufschema von Figur 1 im Einzelnen dargestellt.

In der ersten Stufe, der Hydrolyse, werden Makromoleküle in Monomere und lösliche Oligomere zerlegt. In der anschließenden Acidogenese werden Carbonsäuren, kurzkettige Fettsäuren und Alkohole gebildet. In dieser Stufe entstehen mit Wasserstoff, Kohlendioxid und Essigsäure Edukte der späteren Methanbildung. Im dritten Schritt, der Acetogenese, werden die zuvor gebildeten Alkohole, kurzkettigen Fett- und Carbonsäuren zur Essigsäure bzw. Acetat umgebaut. In der letzten Stufe produzieren Mikroorganismen, die zur Gruppe der sog. Archaeen gehören, aus Wasserstoff und Kohlendioxid (Wasserstoffverwertender Weg oder hydrogenotrophe Methanogenese) gemäß nachfolgender Reaktionsgleichung (1) bzw. aus der Essigsäure (Essigsäure spaltender Weg oder acetoklastische Methanogenese) gemäß nachfolgender Reaktionsgleichung (2) Methan:

hydrogenotrophe Methanogenese:

$$CO_2 + 4\ H_2 \rightarrow CH_4 + 2\ H_2O \qquad (1)$$

acetoklastische Methanogenese:

$$CH_3COO^- + H^+ \rightarrow CH_4 + CO_2 \qquad (2)$$

Das gebildete Methan entweicht gemeinsam mit überschüssigem $CO_2$ aus dem Reaktorgärsubstrat. Die vier Stufen lassen sich dabei nicht strikt voneinander trennen, da beispielsweise auch schon in der Acidogenese Essigsäure und Wasserstoff entstehen.

Es gibt bereits eine große Anzahl von Veröffentlichungen aus dem Stand der Technik in diesem Bereich, von denen hier die Folgenden erwähnt werden sollen:

So beschreibt die DE 34 27 976 A1 ein Verfahren und eine Vorrichtung zur anaeroben Behandlung von Substraten mit organischen Stoffen zur Erzeugung von Biogas. Hierbei wird in einem Zweistufen-Reaktor der Reaktor in 2 Reaktorräume aufgeteilt, wobei im ersten Reaktorraum die Hydrolyse- und Säurebildung und im zweiten Reaktorraum die Methanbildung erfolgt. Um einen größeren Anteil an Methan zu erzeugen und einen hohen Abbaugrad bei kurzer Verweilzeit zu erzielen,

wird eine kontinuierliche Reduzierung des Wasserstoffpartialdrucks und damit die Einstellung des pH-Werts im ersten Reaktor durchgeführt, wodurch ein erhöhter Essigsäureanteil und eine optimale Verteilung an besser abbaubaren Stoffwechselprodukten erfolgen soll.

Die DE 10 2014 006 501 A1 bezieht sich auf ein Verfahren zur Erzeugung von Biogas. Hierbei soll die Wirtschaftlichkeit der Anlage dadurch gesteigert werden, dass die erzeugte Biogasmenge durch eine Veränderung des Mengenstroms der zugegebenen Stoffe so beeinflusst wird, dass unter Berücksichtigung der Stabilität des biologischen Prozesses stets der maximale Gewinn der Anlage erzielt wird. Hierzu werden regelmäßig und in relativ kurzen Zeitabständen eine oder mehrere, die Stabilität des Gärprozesses charakterisierende Kenngrößen bestimmt und automatisiert oder teilautomatisiert berücksichtigt, indem der Mengenstrom an zugeführten organischen Stoffen so angepasst wird, dass die Kenngrößen Werte annehmen, die einem stabilen Gärprozess entsprechen. Der Mengenstrom wird anhand eines empirischen Modells bestimmt. Eine der Kenngrößen ist der FOS/TAC-Wert.

Ein Ansatz zur Prozessintensivierung ist in der DE 10 2010 043 779 A1 beschrieben, wonach zur gezielten Vermehrung von acetogenen Mikroorganismen einer Biogasanlage adaptionsauslösende Substrate zudosiert werden, so dass höhermolekulare Carbonsäuren beschleunigt abgebaut werden.

Weiterhin offenbart die WO 2007/014717 A1 ein Verfahren zur Herstellung von Biogas, wobei Biomasse unter anaeroben Bedingungen vergoren wird und dann im Gegensatz zum üblichen Verfahren, das ohne Sauerstoff betrieben wird, dosierter Eintrag von Sauerstoff bzw. Luft in das Gärsubstrat des Methanbildungsreaktors erfolgt, wodurch die Reaktionsgeschwindigkeit der Vergärungsvorgangs erhöht wird.

Es hat sich herausgestellt, dass für einen sicheren und störungsfreien Betrieb einer Biogasanlage die Prozessstabilität entscheidend ist. Prozessstabilität bedeutet, dass die einzelnen Stufen - Hydrolyse (1. Stufe), Acidogenese (2. Stufe), Acetogenese (3. Stufe) und Methanogenese (4. Stufe) - im Biogasreaktor miteinander im Gleichgewicht stehen. Damit zwischen allen Stufen des anaeroben Abbaus Gleichgewicht vorliegt, sollten möglichst eine gleichmäßige Substratzufuhr, konstante Substratzusammensetzung sowie konstante Gärbedingungen vorliegen. Dies ist jedoch nicht immer möglich.

Das Gleichgewicht des anaeroben Abbaus zwischen den beschriebenen 4 Stufen ist daher entscheidend für die Prozessstabilität und hohe Methanausbeuten. Eine Störung des Gleichgewichts kann durch verschiedene Faktoren, wie Temperaturänderung, Hemmstoffe, Nährstoffmangel, Änderung der Substratzufuhr, z.B. zu hohe Substratzufuhr (Raumbelastung), Änderung der Substratzusammensetzung, Anreicherung von Säuren, Verschlechterung der Lebensbedingungen der methanbildenden Mikroorganismen in der letzten Stufe des Abbaus, eintreten. Insbesondere die Änderung der Substratzufuhr und -zusammensetzung spielen häufig eine Rolle, da Anlagenbetreiber naturgemäß die Biogasproduktion maximieren möchten, was sie durch eine erhöhte Zufuhr an organischer Substanz mit verbesserter Abbaubarkeit zu erreichen versuchen.

Eine weitere Gefahr für die Prozessstabilität ist die flexible Fahrweise vieler Anlagen, die bedarfsgerecht zu bestimmten Tageszeiten oder an bestimmten Wochentagen besonders viel Biogas produzieren und verstromen möchten. Die steigende bzw. variable Raumbelastung (Zufuhr organischer Substanz pro Fermentervolumen und Zeit) und verringerte Verweildauer können zu einer Prozessstörung führen. Dabei entsteht ein Ungleichgewicht zwischen Säureproduktion (erste drei Stufen) und Säureabbau in der Methanogenese (Voß, E., Weichgrebe, D., Rosenwinkel, K.-H. (2009). FOS/TAC: Herleitung, Methodik, Anwendung und Aussagekraft, Internationale Wissenschaftstagung Biogas Science 3, 675 - 682). Die organischen Säuren reichern sich an und durch eine Abnahme des pH-Wertes im Gärsubstrat werden die Lebensbedingungen für die methanogenen Archaeen zunehmend ungünstig. Als Folge nehmen die Methanproduktion und die Methangehalte im Biogas ab. Wenn dieser Zustand länger andauert bzw. unbemerkt bleibt, kann dies bis zu einem kompletten Prozessversagen mit erheblichen wirtschaftlichen Konsequenzen führen, wie mehrwöchiger Produktionsausfall, Entsorgung des Fermeterinhaltes, neues Anfahren der Anlage, etc.

Eine Möglichkeit, einen Anlagestillstand zu vermeiden, ist es, den Anlagenbetrieb mit hohen Sicherheitsreserven durchzuführen, ohne die Potentiale der Anlage wirklich auszuschöpfen. Dies führt aber zu deutlichen Erlösverlusten und daher letztlich einer nicht wirtschaftlichen Betriebsweise, die unerwünscht ist.

Obwohl bereits viele Ansätze für die Prozessüberwachung angewandt und getestet worden sind, ist eine breite Anwendung einer prozessbiologiebezogenen Mess- und Regelungstechnik in der Biogaspraxis bislang nicht möglich (siehe Nguyen, D., Gadhamshetty, V., Nitayavardhana, S., Khanal, S. K. (2015), Automatic process control in anaerobic digestion technology: A critical review, Bioresource Technology, 193, 513-522). Ein Bedarf hierfür wird aber mit fortschreitender Flexibilisierung der Biogasproduktion zunehmen.

Es besteht deshalb verstärkt ein Bedürfnis, Parameter der Prozessstabilität für einen Bioreaktor zu erfassen, diese zu beurteilen und diese Information an den Anlagenbetreiber zeitnah weiter zu geben.

Wie bereits beschrieben, entstehen beim anaeroben Abbau organische Säuren, die nachfolgend abgebaut werden müssen, um den Fermenterinhalt nicht zu übersäuern, da die Gefahr des Stillstands der Methanproduktion besteht. Für einen effizienten Betrieb der Biogasanlage ist es daher unerlässlich, ein Ungleichgewicht zwischen Säureproduktion und Säureabbau möglichst frühzeitig zu erkennen, um entsprechende Gegenmaßnahmen, wie z.B. die Verringerung der Biomassezufuhr, einzuleiten. Daher ist es erforderlich, die Dynamik der mikrobiellen Prozesse und

den Zustand des anaeroben Abbaus durch geeignete Systeme zu überwachen.

Der pH-Wert der Reaktorflüssigkeit würde sich auf den ersten Blick als Überwachungsparameter hierfür anbieten. Die anaeroben Biogasreaktoren arbeiten in einem pH-Wert-Bereich von etwa 7,0 bis etwa 8,2, wo das System durch das vorhandene Hydrogenkarbonat eine sehr hohe Pufferkapazität aufweist. Der pH-Wert des Fermenterinhaltes könnte auch einfach online gemessen werden. Dieser reagiert aber mit starker Verzögerung auf eine übermäßige Säureproduktion und zeigt somit kritische Entwicklungen erst nach dem Aufbrauchen des Puffers an, so dass entsprechende Gegenmaßnahmen nicht rechtzeitig erfolgen können. Daher ist die pH-Messung für eine Beurteilung der Prozessstabilität nicht geeignet. Eine prozessbegleitende (online) Messung des pH-Wertes ist aus diesen Gründen in der Biogaspraxis nicht bzw. nur sehr selten anzutreffen.

Bekannt ist auch, dass hohe Wasserstoffkonzentrationen eine Stoffwechselstörung in der Acetogenese (dritte Stufe des Abbaus) verursachen und eine Propionsäureackumulation anzeigen (Weiland, P. (2010), Biogas production: current state and perspectives, Applied Microbiology and Biotechnology, 85(4), 849-60). Aus diesem Grund wurde versucht, die Wasserstoffkonzentration im flüssigen Reaktorinhalt zu bestimmen. Experimentelle Gärversuche haben zwar hohe Sensitivität des benutzten Messsystems gezeigt (Boe, K., Batstone, D. J., Steyer, J.-P., Angelidaki, I. (2010), State indicators for monitoring the anaerobic digestion process, Water Research, 44(20), 5973-80), aufgrund der Gerätekomplexität ist die Sensorik über einen experimentellen Ansatz bei der Überwachung der Prozessstabilität bislang jedoch nicht hinausgekommen.

Derzeit werden daher überwiegend zwei Methoden zur Beurteilung der Prozessstabilität verwendet:
Die erste Methode ist die Bestimmung der Konzentration kurzkettiger organischer Fettsäuren; die zweite Methode ist die Bestimmung des sogenannten FOS/TAC-Werts (Voß, E.,2009 et al. a.a.O., Nguyen, D. 2015 et al. a.a.O.).

Gemäß der ersten Methode wird die Konzentration kurzkettiger organischer Fettsäuren (Essig-, Propion- und Buttersäure) im flüssigen Reaktorinhalt mittels (Gas-)Chromatographie bestimmt. Hohe Konzentrationen von Essig- und Propionsäure und die Verschiebung ihres Verhältnisses zugunsten der Propionsäure zeugen von einer hohen Prozessbelastung. Diese Messungen erlauben eine sehr gute Prozessbeurteilung, bleiben jedoch wegen aufwendiger Probenvorbehandlung und Gerätebedienung spezialisierten Analyselabors vorbehalten. Die Kosten für eine Gaschromatographie-Anlage sind zudem hoch und ihre qualifizierte Wartung und Bedienung sind in der Biogaspraxis daher nicht möglich, so dass der Anlagenbetreiber auf ein entsprechendes Labor angewiesen ist. Seitens des Anlagenbetreibers werden die Proben dorthin versandt und die Ergebnisse liegen innerhalb von Tagen vor. Aus diesen Gründen scheidet diese Methode für eine automatische Überwachung der Stabilität der Biogasproduktion aus. Insbesondere der zeitliche Faktor, wonach Tage zwischen Probenahme und erhaltenem Ergebnis liegen, zeigt die begrenzte Einsetzbarkeit des Verfahrens auf. Die Bestimmung organischer Säuren durch Gaschromatographie ist in der Biogaspraxis vor Ort daher praktisch nicht einsetzbar.

Anwendungen, welche die Fettsäurenkonzentration direkt im Fermenterinhalt bestimmen (siehe Boe, K. et al., a.a.O. und Nielsen, H. B., Uellendahl, H., Ahring, B. K. (2007), Regulation and optimization of the biogas process: Propionate as a key parameter, Biomass and Bioenergy, 31(11-12), 820-830), bedürfen ebenfalls einer aufwendigen Probenvorbehandlung, wie Filterung, Zentrifugation, etc., was eine vor Ort-Messung in einer Biogasanlage kostspielig und störungsanfällig macht und einen weiten praktischen Einsatz ausschließt. Stockl und Öchsner (Stockl, A., Oechsner, H. (2012), Near-infrared spectroscopic online monitoring of process stability in biogas plants, Engineering in Life Sciences, 12(3), 295-305) haben den Einsatz drei gekoppelter NIRS-Sensoren im Pilotmaßstab vorgeschlagen und hohe Kalibrierwerte zwischen den Säurekonzentrationen und den NIRS-Werten erreicht; allerdings sind die Kosten der NIRS-Systeme noch relativ hoch. Weiterhin hat die Komplexität solcher Messeinrichtungen bislang zu keiner breiten Anwendung in der Biogaspraxis geführt (Nguyen et al. 2015, a.a.O.).

Gemäß der zweiten Methode wird der FOS/TAC-Wert bestimmt, der als Indikator der Prozessstabilität am weitesten verbreitet ist. In dieser Verhältniszahl gibt der FOS-Wert den Gehalt an <u>F</u>lüchtigen <u>O</u>rganischen <u>S</u>äuren an und der TAC-Wert steht für <u>T</u>otales <u>A</u>lkalines <u>C</u>arbonat und bildet das Puffervermögen ab (Voß et al. 2009, a.a.O.). Es ist ein titrimetrischer Vergleich der Gesamtsäuren und der Pufferkapazität. Die meisten Arbeiten geben an, dass der FOS/TAC-Wert bei einem stabilen Prozess unterhalb von 0,4 liegen sollte. Hohe FOS/TAC-Werte sind ein Hinweis für eine zu hohe Konzentration an organischen Säuren bei gleichzeitiger Erschöpfung des Puffervermögens. Zu niedrige Werte, beispielsweise unterhalb von 0,1, deuten auf eine "Unterlast" hin. Eine solche Anlage hat das Potenzial, mehr Substrat umzusetzen.

Der FOS/TAC-Wert wird über eine zwei-Stufen-Titration an einer Probe des Fermenterinhaltes ermittelt. Dabei wird eine Probe der Fermenterflüssigkeit entnommen und titriert. Hierzu wird ein pH-Meter, verbunden mit einem Titrierautomat und einer Dosiereinrichtung für die Säure, verwendet. Zunächst wird hierbei mit 0,1 M Schwefelsäure der TAC-Wert durch eine Titration der Probe von ihrem bestehenden pH-Wert auf einen pH-Wert von 5,0 ermittelt. Um den Gehalt an flüchtigen organischen Säuren (FOS-Wert) zu bestimmen, wird analog dem TAC-Wert die Probe weiter von pH 5 zu pH 4,3 titriert, da die Protonen in diesem pH-Bereich von den organischen Säuren aufgenommen werden. Nach der FOS/TAC-Bestimmung wird die titrierte Probe verworfen.

In der Biogaspraxis wird die FOS/TAC-Bestimmung in sehr unterschiedlichen Intervallen, insbesondere wöchentlich bis monatlich, vorgenommen, da dies mit hohem zeitlichen (Entnahme und Versand der Probe) und finanziellen Aufwand verbunden ist. Die beschriebene zweistufige Titration übersteigt meist die Möglichkeiten von Biogasanlagen in der Praxis. Zudem gehen bei Lagerung und Transport die mikrobiellen Prozesse weiter, so dass die spätere FOS/TAC-Bestimmung mit Fehlern behaftet sein kann. Dies erlaubt nicht, auf veränderte Substratzufuhr und -eigenschaften unmittelbar zu reagieren und die Anlage auf eine stabile Höchstleistung zu optimieren. In Labortests mit einem wünschenswerten Entnahmeintervall von Stunden bis Tagen kann die FOS/TAC-Bestimmung die Prozessstabilität zeitlich hochauflösend nicht abbilden, da eine häufige Entnahme der jeweils ca. 20-40 ml Probe über die Verringerung des Fermenterinhaltes zu einer Verfälschung der Ergebnisse bzw. der berechneten Biogaserträge führt. Eine Automatisierung ist somit nicht wirklich umsetzbar.

Wie bereits dargelegt, besteht aber ein großer Bedarf an einer online-Erfassung der Prozessstabilität von Biogasanlagen, um den höchstmöglichen Methanertrag aus den zugeführten Substraten und dem verfügbaren Fermentervolumen zu erzielen. Dies können die oben geschilderten beiden Methoden nicht bereitstellen. Zusätzlich verstärkt wird dieser Bedarf durch den Übergang vieler Biogasanlagen mit konstanter Leistung in die flexible Stromproduktion. Eine stoßweise Substratzufuhr, um zeitgerecht über zusätzliche Biogasmengen zu verfügen, fördert insbesondere die ersten Stufen des anaeroben Abbaus und kann die Prozessstabilität zusätzlich belasten, wie bereits erläutert wurde. Aus diesen Gründen ist der Bedarf an einer automatisierten Anlageüberwachung aktuell sehr hoch und auch wirtschaftlich vorteilhaft.

Auch zur automatisierten Überwachung von Biogasanlagen gibt es eine Reihe an Veröffentlichungen aus dem Stand der Technik. Beispielhaft werden die folgenden angeführt und kurz erläutert:
Eine Möglichkeit für eine automatisierte Anlagenüberwachung wird beispielsweise in der EP 2 078 947 A2 offenbart, die eine Messvorrichtung und ein Messverfahren zur automatisierten Messung der Eigenschaften des in einer Biogasanlage befindlichen Faulschlamms beschreibt. Die Methode basiert analog zum TAC-Wert auf der Erfassung des Hydrogenkarbonats, das die Pufferleistung des Systems abbildet. In einem Messzylinder wird aus der entnommenen Probe des Biogasreaktors das gelöste Hydrogenkarbonat durch Salzsäurezugabe freigesetzt. Anschließend wird mittels einer Gasmengenerfassungsvorrichtung die Menge des entstandenen Gases bestimmt. Da dieser Ansatz die Fettsäurebelastung des Prozesses nicht erfasst, ist er der FOS/TAC-Bestimmung von der Aussagekraft her unterlegen und obendrein technisch sehr komplex durchzuführen.

Ferner wird in der in DE 10 2016 013 068 A1 eine automatisierte FOS/TAC-Bestimmung in Biogasanlagen zur anaeroben Nassvergärung von organischen Stoffen offenbart. Hierbei wird eine Bypass-Leitung aus dem Biogasreaktor als Dosiereinrichtung verwendet, die zur Entnahme einer repräsentativen Probe dient. Bei der Probengewinnung wird diese Leitung an zwei Stellen (3-Wege-Armatur) automatisch geschlossen und eine Probe abgezweigt. Durch eine nachfolgend angeordnete Abscheidevorrichtung werden die festen Bestandteile weitgehend abgetrennt und die erhaltene dünnflüssige Probe mit destilliertem Wasser verdünnt. Anschließend wird mit verdünnter Schwefelsäure pH-Wert-gesteuert titriert. Eine Steuereinrichtung berechnet aus den verbrauchten Säuremengen den FOS/TAC-Wert und gibt diesen über eine Schnittstelle aus. Die titrierte Probe wird danach in ein Abwassersystem entleert und die ganze Einheit mittels einer Spüleinrichtung gereinigt. Das in der DE 10 2016 013 068 A1 angegebene Verfahren ist daher sehr komplex. Es muss eine Bypass-Leitung eingerichtet werden und es werden zum Beispiel Ventile, Spüleinrichtungen, Abwasserzugänge, Vorratsgefäße für Schwefelsäure und destilliertes Wasser benötigt. Dadurch beinhaltet diese automatisierte FOS/TAC-Bestimmung eine ganze Reihe potentieller Probleme. Dies betrifft beispielweise das automatische Öffnen und Schließen der 3-Wege-Ventile, Mengenfehler bei der Entnahme, Totvolumina der Leitungen, automatisches Befüllen und Entnahme des Probeninhaltes der Zentrifuge sowie ihre Reinigung, Dosiereinrichtungen für Probe und Verdünnungswasser, Reinigung der Abscheidevorrichtung durch Rückspülung etc. Durch den hohen Installationsaufwand und die technische Komplexität dieses vorgeschlagenen Verfahrens ist eine praxisnahe Verwendung in Biogasanlagen nicht bzw. kaum möglich.

In der WO 2014/128300 A1 wird ein Verfahren zur Herstellung von Methan beschrieben, umfassend das Kontaktieren von methanogenen Mikroorganismen in einem Reaktionsgefäß mit Wasserstoff und Kohlendioxid, wobei die Stickstoffkonzentration in der Flüssigphase innerhalb des Reaktionsgefäßes im Bereich von 0,0001 bis 35 mMol/L oder im Bereich von 55 bis 1000 mMol/L liegt. Die Bestimmung der Stickstoffkonzentration dient dazu, eine möglichst hohe Methanausbeute zu bekommen. Weiterhin wird der $CO_2$-Partialdruck im gasenthaltenden Kopfraum des Reaktors bzw. in der Vorrichtung, in der das Prozessabgas aus dem Reaktor entfernt wird, gemessen.

Schließlich bezieht sich die DE 10 2011 110 638 A1 auf ein Verfahren zur Regelung einer Biogasanlage, wobei die Zufuhr von Substraten in einen Fermenter (1) und einen Nachgärer (2) der Biogasanlage durch ein Regelungssystem (3) im laufenden Betrieb der Biogasanlage geregelt wird, dem als Eingangsgröße (4) eine durch Computersimulation bestimmte Substratzufuhrstrategie zugefügt wird, die nach Maßgabe der verfügbaren Substrate bestimmt wird. Dies ist ebenfalls ein sehr komplexes Mess- und Regelverfahren für Biogasanlagen, wobei eine Vielzahl von Parametern Berücksichtigung findet.

Gemäß einem weiteren Aspekt, der für die vorliegende Erfindung relevant ist, kann eine Biogasanlage auch zur Stromspeicherung eingesetzt werden:

Die im Rahmen der Energiewende zunehmende Stromproduktion aus Wind- und Solarkraftwerken (Photovoltaik-Anlagen) führt zu Produktionsspitzen, die im gesamten Energiesystem abgefangen werden müssen. Aktuell wird in Deutschland bei neu installierten Photovoltaik-Anlagen eine Drosselung auf 60 bzw. 70 % der Peakleistung voreingestellt. Bei größeren Anlagen steuert der Netzbetreiber die aktuelle Leistung der Anlage. Ebenso müssen Windkraftwerke teilweise abgeregelt werden, da sonst die Netzstabilität gefährdet sein könnte. In den kommenden Jahren wird die weiter steigende Stromproduktion aus Wind- und Solarkraftwerken diese Problematik verschärfen.

In Deutschland werden im kommenden Jahrzehnt viele der derzeit im Betrieb befindlichen ca. 9.000 Biogasanlagen nach dem Auslaufen der EEG-Förderung über die reine Stromproduktion kaum mehr wirtschaftlich betrieben werden können.

Hierfür wurde das energiewirtschaftliche "Power-to-Gas-Konzept" (abgekürzt als "PtG") entwickelt, bei dem überschüssiger Strom aus erneuerbaren Energiequellen dazu verwendet wird, durch Wasserelektrolyse Wasserstoff zu produzieren. Dieser Wasserstoff kann in einem zweiten Schritt unter Verwendung von Kohlendioxid ($CO_2$) zu Methan umgewandelt werden Eine andere Option der PtG-Technologie, den Wasserstoff direkt als Energieträger zu speichern und zu verwenden, ist technisch sehr aufwändig, so dass die Speicherung von Methan im Erdgasnetz als eigentliche Option bleibt. Es ist daher vorstellbar, dass für Biogasanlagen das biologische Power-to-Gas-Verfahren ein Weg zu mehr Rentabilität sein wird, zumal das regenerativ produzierte "Bio-Erdgas" an Akzeptanz und Bedeutung zunehmen wird. Mit einem weiter zunehmenden Anteil an regenerativem Strom wird die Notwendigkeit der Energiespeicherung stark ansteigen und somit ist mit einem breiten Einsatz des biologischen PtG-Verfahrens zu rechnen.

Die Power-to-Gas-Konzepte sind ein Weg, wie die überschüssige Energie abgefangen und im Erdgasnetz gespeichert werden kann. Es stehen hierzu zwei PtG-Verfahren zur Verfügung: (1) das katalytische Verfahren und (2) das biologische Verfahren (Götz, M., Lefebvre, J., Mörs, F., Koch, A.M., Graf, F., Bajohr, S., Reimert, R., Kolb, T. (2016), Renewable Power-to-Gas: A technological and economic review, Renewable Energy 85, 1371-1390). Die nachfolgende biologische Methanisierung wird auch als biologische Wasserstoff-Methanisierung bezeichnet.

Die biologische Methanisierung kann entweder *in situ,* d.h. in einem bestehenden Biogasreaktor, in den zusätzlich Wasserstoff eingeblasen wird, oder *ex situ* in einem separaten Reaktor, der mit beiden Gasen beaufschlagt wird, erfolgen. In beiden Verfahren setzen methanogene Archaeen Wasserstoff und Kohlendioxid zum Methan um, d.h. es finden die bereits geschilderten Reaktionen statt, die bei der Erzeugung von Biogas in Biogasanlagen erläutert wurden.

In beiden PtG-Verfahren liegen Kohlendioxid und Wasserstoff zweckmäßigerweise stöchiometrisch im Verhältnis von 1:4 vor. Dies ist insbesondere in der biologischen Wasserstoff-Methanisierung bei der Nutzung eines getrennten Methanisierungsreaktors erforderlich. Bei der *in situ*-Methanisierung wird meist nur Wasserstoff zudosiert und ggf. auch das entstandene methanreiche Biogas mit bis zu 10 % Wasserstoff rezirkuliert, um die Methangehalte weiter zu erhöhen und den Anteil an Wasserstoff im Produkt zu reduzieren.

Es gibt hierzu ebenfalls zahlreiche Veröffentlichungen, von denen hier einige herausgegriffen werden sollen: Beispielweise wird in der DE 10 2011 015 415 A1 die biologische Methanisierung im *ex situ* Verfahren in getrennten Methan-Druck-Reaktoren realisiert. In einem vorgeschalteten Biogasreaktor wird eine Perkolatflüssigkeit durch anaeroben mikrobiellen Abbau von Biomasse hergestellt. Im nachfolgenden Druckreaktor wird im Perkolat über eine Druckerhöhung auf bis zu 100 bar die Verfügbarkeit von $H_2$ und $CO_2$ für die Mikroorganismen gesteigert. Die Verfügbarkeit von $CO_2$ wird hier nicht berücksichtigt und nicht überprüft.

In der DE 10 2009 053 593 A1 ist ein Verfahren zur Steigerung des Leistungspotentials von Biogasanalgen durch $H_2$-Zudosierung beschrieben. Hierbei wird auch eine optionale $H_2$-abhängige Steuerung mittels $H_2$-Sensoren hinsichtlich einer maximalen Methanbildungsrate beansprucht. Eine $CO_2$-abhängige Steuerung zur Erzielung derselben wird jedoch nicht offenbart.

Weiterhin bezieht sich die DE 10 2014 103 311 A1 im Bereich der Methanisierung u.a. auf die Nutzung der Kraftwerksabgase, um diese mit einer Methanisierungsanlage zu koppeln. Es ist u.a. die $CO_2$-Aufbereitung und die Abwärmenutzung der Methanisierungsanlage beschrieben; eine Kontrolle des $CO_2$-Partialdruckes im Methanisierungsprozess ist aber nicht vorgesehen.

Gemäß der Offenbarung der WO 2013/060331 A1 wird die Wasserstoffzudosierung zur Erhöhung der Methangehalte im Biogas (biogas upgrading) beschrieben. Hierbei wird $H_2$ über eine permeable Membran zugeführt. Die Prozessparameter wurden laboranalytisch bestimmt (TOC, GC-TCD). Aus den Analysedaten wurden dann die anorganischen Kohlenstoffspezies (darunter auch $CO_2$) in der Flüssigkeit des Reaktors berechnet. Die erforderlichen Laboranalysen des Fermenterinhaltes schließen jedoch eine Prozesskontrolle in Echtzeit aus. Ebenso wurden die Konzentrationen der Kohlenstoffspezies nicht als Prozessparameter betrachtet.

Ferner beziehen sich die DE 10 2016 000 070 A1 und die WO 2018/108810 A1 auf einen Methanisierungsreaktor, in dem an einer anaerob-bioreaktiven permeablen Wand methanogene Archaeen die gasförmig zugeführten Edukte $CO_2$ und $H_2$ zu $CH_4$ umwandeln. Hierbei wird in der DE 10 2016 000 070 A1 auch die Konzentration der Edukte auf der Produktseite gemessen und diese Information zur Regelung des Differenzdruckes auf beiden Seiten der Reaktionswand eingesetzt (s. Patentanspruch 2). Diese Anwendung unterscheidet sich jedoch von der vorliegenden Erfindung

insofern, dass in der DE 10 2016 000 070 A1 nur außen in der Gasphase des Reaktors die $CO_2$-Konzentration gemessenen wird, die biologische Methanisierung aber in der Flüssigphase stattfindet, wofür der dort auftretende $CO_2$-Partialdruck entscheidend wäre.

Ein Problem bei der *in situ*-Methanisierung ist, die Verfügbarkeit von $CO_2$ in der Flüssigphase des Reaktors, die - wie auch durch die oben angegebenen Veröffentlichungen gezeigt - regelmäßig nicht überwacht wird. Unter diesen Umständen kann die angestrebte intensive hydrogenotrophe Methanbildung den $CO_2$-Pool im Reaktor so weit verringern, dass es die weitere Methanogenese beeinträchtigt. Ebenfalls können ein Anstieg des pH-Wertes und die Akkumulation von Fettsäuren den gesamten Prozess stören.

Bei der *in situ*-Methanisierung wird der Wasserstoff zudosiert, während $CO_2$ aus der mikrobiellen Umsetzung des Biogassubstrates, aber auch aus dem Karbonatpuffersystem, entstammt. Intensive hydrogenotrophe Methanbildung führt dabei zur Erschöpfung des $CO_2$-Pools im Biogassubstrat, was zu einem Anstieg des pH-Wertes auf bis zu pH 9 führt. In der Folge werden die methanogenen Mikroorganismen inhibiert und die flüchtigen Fettsäuren reichern sich an, was zu einer Destabilisierung des gesamten Prozesses führt (Agneessens, L.M., Ottosen, L.D.M., Ottosen, N.V., Nielsen, J.L., Jonge, N., Fischer, Ch. H., Kofoed, M.V.W. (2017), In-situ biogas upgrading with pulse H2 additions: The relevance of methanogen adaption and inorganic carbon level, Bioresource Technology 233, 256-263).

Es treten demnach sowohl bei der Biogaserzeugung als auch beim biologischen Methanisierungsverfahren Probleme hinsichtlich der Prozessstabilität auf.

Der Erfindung liegt daher die Aufgabe zugrunde, die Nachteile aus dem Stand der Technik zu überwinden und ein Verfahren bereitzustellen, das eine Überwachung der Prozessstabilität der im Biogasreaktor ablaufenden Reaktionen ermöglicht, so dass diese Information dem Anlagenbetreiber zeitnah zugänglich ist. Das Verfahren soll ohne großen technischen Aufwand und in relativ kostengünstiger Art und Weise in Biogasanlagen jeder Art und Größe in der Praxis in einfacher Weise verwirklichbar sein und eine automatische Überwachung der Biogasanlage ermöglichen.

## Kurze Beschreibung der Erfindung

Die vorliegende Erfindung löst die oben geschilderte Aufgabe durch ein Verfahren zum automatischen Überwachen der Stabilität der Methanerzeugung in einem oder mehreren Biogasreaktoren zur anaeroben Vergärung von organischer Substanz mit den Schritten:

- Bestimmen des $CO_2$-Partialdrucks ($pCO_2$) im flüssigen Reaktorinhalt unter anaeroben Messbedingungen mit einem Messsystem während der Biogaserzeugung, wobei die $pCO_2$-Messwerte automatisch in regelmäßigen einstellbaren Zeitabständen ermittelt werden, und
- Einsetzen der ermittelten Werte zur prozesstechnischen Bewertung als Parameter zur Stabilitätsbeurteilung des Prozesses der Biogas- und Methanbildung. Gegenstand der Erfindung ist auch ein Verfahren zum automatischen Überwachen der Stabilität der Methanerzeugung in einem oder mehreren Biogasreaktoren zur anaeroben Vergärung von organischer Substanz, wobei die Methanerzeugung eine biologische Methanisierung, insbesondere eine biologische Methanisierung im *in* situ-Verfahren in einem Power-to-Gas-Verfahren, darstellt, wobei der organischen Substanz im anaeroben Reaktor zusätzlich Wasserstoff und gegebenenfalls auch Kohlendioxid zugeführt werden, mit den Schritten:

    - Bestimmen des $CO_2$-Partialdrucks ($pCO_2$) im flüssigen Reaktorinhalt unter anaeroben Messbedingungen mit einem Messsystem während der Biogaserzeugung, wobei die $pCO_2$-Messwerte automatisch in regelmäßigen einstellbaren Zeitabständen ermittelt werden, und
    - Einsetzen der ermittelten Werte zur prozesstechnischen Bewertung als Parameter zur Stabilitätsbeurteilung des Prozesses der Biogas- und Methanbildung.

Das erfindungsgemäße Verfahren findet damit sowohl bei der Methanerzeugung in einer Biogasanlage als auch bei der biologischen Methanisierung Anwendung. Das beschriebene Verfahren könnte auch in der anaeroben (Methan-)Stufe der Abwasserreinigung sowie in allen anaeroben Prozessen mit dem Ziel der Methanerzeugung eingesetzt werden.

Bei der biologischen Methanisierung wird eine Biogasanlage oder ein externer Reaktor zur Erzeugung von Methan eingesetzt, jedoch wird zusätzlich Wasserstoff und gegebenenfalls auch Kohlendioxid zum Reaktorinhalt zugeführt. Im Einzelnen wandeln daher auch bei der biologischen Methanisierung hochspezialisierte Mikroorganismen, sog. Archaeen, biokatalytisch die Verbindungen Wasserstoff ($H_2$) und Kohlendioxid ($CO_2$) zu Methan ($CH_4$) um. Die mikrobiellen Stoffwechselprozesse laufen ebenfalls unter strikt anaeroben Bedingungen und in einer wässrigen Umgebung unter Verwendung von Gärsubstrat ab und wurden bereits im Einzelnen beschrieben. Im Gegensatz zum *ex situ*-Verfahren, bei dem die biologische Methanisierung in einer separaten Methanisierungsanlage erfolgt, wird beim *in situ*-Verfahren der Wasserstoff für die biologische Methanisierung direkt in das Gärmaterial des Fermentationsprozesses gegeben. Die biologische Methanisierung *in situ* wird daher in bestehenden, aus dem Stand der Technik bekannten Biogasreaktoren angewandt. Dabei erfolgt die Biogasbildung aus dem zugeführten organischen Substrat, unterstützt durch die zusätzliche $H_2$-Einbringung. Bei der Zudosierung von $H_2$ wird das im flüssigen Reaktorsubstrat vorhandene $CO_2$ von den methanbildenden Archaeen verbraucht. Das aus der anaeroben Umsetzung dieses Substrats entstandene $CO_2$ (siehe Figur 1, Schritt 2 und 3) wird zusammen mit dem extern zugeführten $H_2$ in der hydrogenotrophen Methanogenese zu $CH_4$. Bei der vorliegenden Erfindung ist das *in situ*-Verfahren bevorzugt. Die biologische Methanisierung spielt eine große Rolle für die Speicherung von Stromüberschüssen bei erneuerbaren Energien.

Auch in der biologischen Methanisierung ist die Erfassung des verfügbaren $CO_2$-Pools im Substrat über die Messung des $CO_2$-Partialdrucks im flüssigen Reaktorinhalt bislang nicht bekannt geworden. Die bisherigen PtG-Verfahren messen derzeit den $pCO_2$ in der Flüssigkeit ihrer Reaktoren nicht. Es hat sich jedoch herausgestellt, dass dies ein unverzichtbarer Bestandteil des biologischen PtG-Verfahrens sein sollte. Wenn die $CO_2$-Verfügbarkeit nicht überwacht wird, kann es zu einer Erschöpfung des $CO_2$-Pools kommen, womit der Methanisierungsprozess ineffizient bzw. instabil wird. Die mangelnde Überwachung der $CO_2$-Verfügbarkeit kann einer der Gründe sein, warum die biologische Methanisierung bislang kaum über den Pilotmaßstab hinausgekommen ist. Wenn - wie durch die beschriebene Erfindung möglich - die $CO_2$-Verfügbarkeit erfasst und durch eine gezielte Zudosierung von $CO_2$ optimiert werden kann, kann eine konstant hohe Methanbildungsrate erreicht werden.

Die biologische Methanisierung erfordert daher begleitend zu einer $H_2$-Zudosierung eine ständige Kontrolle des verfügbaren $CO_2$-Pools im Biogassubstrat. Dies kann effektiv über die laufende Überwachung des $CO_2$-Partialdrucks im flüssigen Reaktorinhalt erfolgen. Die vorliegende Erfindung stellt daher auch einen wesentlichen Baustein für eine effiziente und breite Anwendung des biologischen PtG-Verfahrens dar.

Die Ausführungen in der gesamten Beschreibung beziehen sich daher gleichermaßen auf beide Verfahren: die Methanerzeugung in einer Biogasanlage und die biologische Methanisierung, sofern nichts anderes angegeben wird, auch wenn nicht explizit hierauf verwiesen wird.

Das Verfahren der Erfindung bezieht sich demnach - bei Methanerzeugung als auch biologischer Methanisierung - auf eine direkte Messung des $CO_2$-Partialdrucks ($pCO_2$) im flüssigen Inhalt des anaeroben Bioreaktors, der in kurzen Zeitabständen automatisch ermittelt wird. Die ermittelten Werte geben Aufschluss über die Prozessstabilität der Biogas- und Methanbildung, so dass der Anlagenbetreiber bei sich andeutenden Problemen hinsichtlich der Stabilität des Verfahrens sofort reagieren kann.

Die ermittelten Werte des $CO_2$-Partialdrucks werden zur prozesstechnischen Bewertung als Parameter für die Prozessstabilität der Biogas- und Methanbildung herangezogen, d.h. diese werden beispielweise zu einem angenommenen oder vorgegebenen Wertebereich in Bezug gesetzt und Abweichungen hierzu bestimmt. Der angenommene oder vorgegebene Wertebereich stellt dabei beispielweise den optimalen Wertebereich dar, in dem die Werte des $CO_2$-Partialdrucks bevorzugt liegen sollen, wenn das Verfahren stabil abläuft. Die optimalen Werte können sich dabei in gewissen Grenzen von Bioreaktor zu Bioreaktor unterscheiden und hängen von einer Vielzahl von Parametern, wie z.B. der Prozesstemperatur, Einbautiefe des Messystems, etc. ab. Man kann beispielsweise von Standard-Werten oder Standard-Wertebereichen ausgehen, die durch den Fachmann für jeden Anwendungsfall entsprechend modifiziert werden können. Zur Bestimmung eines geeigneten Wertebereichs kann der Fachmann zum Beispiel eine Reihe an orientierenden Messungen in der betreffenden Anlage durchführen.

Die Bewertung der ermittelten Werte, beispielweise durch einen Vergleich mit einem gewünschten Wertebereich, sorgt dann für eine optimale Prozessführung. Auf Basis der ermittelten $CO_2$-Partialdruck-Werte kann dann in die Anlagensteuerung gezielt manuell, teil- oder vollautomatisiert eingegriffen werden und beispielweise die Zufuhr des Gärsubstrates entsprechend ausgestaltet werden. Dies sorgt unter Wahrung der Prozessstabilität für eine bestmögliche Ausnutzung des Reaktorvolumens. Dadurch können die Biogasanlagen eine bessere Wirtschaftlichkeit erreichen und sind für die Einführung einer flexiblen Biogaserzeugung vorbereitet.

Die vorliegende Erfindung liefert demnach erstmalig ein Verfahren zum automatischen Überwachen einer Biogasanlage, bei dem der $pCO_2$ in der Flüssigphase eines Biogasreaktors während der Biogaserzeugung als Parameter zur Stabilitätsbeurteilung der Biogaserzeugung verwendet werden kann.

**Kurze Beschreibung der Zeichnungen**

Die geschilderten und andere Aspekte, Vorteile und Merkmale gemäß der vorliegenden Erfindung werden in den nachfolgenden Abschnitten auch anhand der beigefügten Zeichnungen in näheren Einzelheiten beschrieben. In den Zeichnungen zeigt:

Figur 1 in schematischer Abbildung die Prozesse beim anaeroben Abbau organischer Substanz in einem Biogasreaktor anhand eines Ablaufschemas (die gestrichelten Pfeile beziehen sich auf Prozesse, welche durch die vorliegende Erfindung abgebildet werden.);

Figur 2 die relative Konzentration verschiedener Kohlenstoffverbindungen in einem Biogasreaktor, aufgetragen gegen den pH-Wert (Schraffur markiert den Bereich der Biogas- und Methanbildung) (nach Lower, S. K. Carbonate equilibria in natural waters, A Chem1 Reference Text, http://citeseerx.ist.psu.edu/viewdoc/ summary?doi=10.1.1.214.2947, modifiziert; siehe auch Stumm, W. und Morgan J.J. (2012), Aquatic Chemistry: Chemical Equilibria and Rates in Natural Waters, 3. Ausgabe, Wiley, 1040 Seiten, Kapitel 4: Dissolved Carbon Dioxide und Libes, S. (2009): Introduction to Marine Biogeochemistry, Elsevier, 2. Ausgabe, 928 Seiten);

Figur 3 eine vereinfachte Darstellung der Quellen und Senken bei der Biogaserzeugung, um die Verwendung der Messung des $CO_2$-Partialdrucks im flüssigen Reaktorinhalt als Verfahren zur Prozessüberwachung zu veranschaulichen;

Figur 4 eine beispielhafte Ausführungsform einer schematisch, vereinfachten Abbildung eines Bioreaktors, in dem ein Messsystem zum Messen des $CO_2$-Partialdrucks im flüssigen Reaktorinhalt gemäß einer Ausführungsform der vorliegenden Erfindung dargestellt ist;

Figur 5 eine beispielhafte Ausführungsform einer schematisch, vereinfachten Abbildung eines Bioreaktors, der zur biologischen Methanisierung eingesetzt wird, wobei ein Messsystem zum Messen des $CO_2$-Partialdrucks im flüssigen Reaktorinhalt gemäß einer Ausführungsform der vorliegenden Erfindung vorliegt;

Figur 6 veranschaulicht den Zusammenhang zwischen den $CO_2$-Partialdruck-Werten ($pCO_2$ in [hPa]) und der Zufuhr von Substrat bzw. der Reduktion von Substrat, durchgeführt im Laufe einer Woche in einer Biogasanlage;

Figur 7 veranschaulicht den Zusammenhang zwischen den $CO_2$-Partialdruck-Werten ($pCO_2$ in [hPa]) und der Zufuhr von Substrat in einer Biogasanlage in einem Langzeittest;

Figur 8 zeigt Prozessstörungen in einer Biogasanlage, dargestellt anhand der FOS/TAC-Werte, der Menge an Gesamtsäuren ([mg/0,1 L]) sowie der $CO_2$-Partialdruck-Werte ($pCO_2$ in [hPa]) im flüssigen Reaktorinhalt und

Figur 9 veranschaulicht den Zusammenhang zwischen den FOS/TAC-Werten und dem $CO_2$-Partialdruck ($pCO_2$ in [hPa]) im flüssigen Reaktorinhalt einer Labor-Biogasanlage.

**Terminologie und Definitionen**

Der Begriff "Biogas" ist hier als das Produkt zu verstehen, das als Folge des anaeroben biologischen Abbaus von organischer Masse in einer Biogasanlage oder einem Biogasreaktor gebildet wird. Biogas enthält dabei üblicherweise ca. 50 bis 60% Methan, 40 bis 50% Kohlendioxid, geringe Mengen an Stickstoff, Schwefelwasserstoff und andere Spurengase sowie Wasserdampf. Methan ist der eigentliche Energieträger.

Als "organische Substanz" oder auch "Biomasse" wird hier die zur Biogas-Herstellung zugrundeliegende Masse bezeichnet, die aus organischer Substanz von Pflanzen oder Tieren bzw. ihrer Exkremente oder aus Abfallprodukten besteht. Die "organische Substanz" bildet zusammen mit der flüssigen Phase, in der Regel hauptsächlich Wasser, den "Reaktorinhalt" oder "Fermenterinhalt" und wird auch als "Gärsubstrat" oder "Maische" bezeichnet. Die "organische Substanz", wie hier verwendet, soll möglichst weitgehend verstanden werden und jede Art von Material umfassen, die in einem Biogasreaktor zur Erzeugung von Biogas eingesetzt werden kann. Es kann sich auch um Gemische aus mehreren Materialien handeln.

"Anaerobe Bedingungen" sind Reaktionsbedingungen im Bioreaktor, die durch die Abwesenheit von freiem oder gelöstem Sauerstoff gekennzeichnet sind. Die anaeroben Bedingungen sind erforderlich wegen der Sauerstoff-Empfindlichkeit der methanbildenden Mikroorganismen, der sog. Archaeen.

Unter "anaerober Vergärung" werden hier energieliefernde, organisches Material zersetzende Stoffwechsel-Prozesse verstanden, die unter anaeroben Bedingungen stattfinden.

Der "Reaktorinhalt" bezieht sich vorliegend nur auf die im Bioreaktor vorliegende organische Substanz oder Biomasse und die vorliegende Flüssigkeit, üblicherweise im Wesentlichen Wasser, d.h. die Fest- und Flüssigphasen-Mischung im Bioreaktor. Die Gasphase im Bioreaktor ist hierbei nicht umfasst und wird - falls diese eine Rolle spielt - gesondert angeführt.

Der Ausdruck "Bestimmen des $CO_2$-Partialdrucks ($pCO_2$) im flüssigen Reaktorinhalt" bedeutet, dass der Partialdruck des Kohlendioxids im flüssigen Reaktorinhalt, genauer gesagt in der flüssigen Phase des Reaktorinhalts bestimmt wird, wobei in der flüssigen Phase, die im Wesentlichen aus Wasser besteht, außerdem die festen Bestandteile, wie z.B. Grassilage und Maissilage, organische Reststoffe und dergleichen, aufgeschlämmt vorliegen. Der Partialdruck des $CO_2$ wird daher in der flüssigen Phase bei gleichzeitiger Anwesenheit der festen Phase, die dispergiert in der

flüssigen Phase vorliegt, ermittelt.

Die Begriffe "Reaktor" und "Fermenter" werden synonym verwendet. Der "Fermenterinhalt" hat demnach die unter "Reaktorinhalt" angeführte Bedeutung.

Das "Reaktorvolumen" oder "Fermentervolumen" bezeichnet den Rauminhalt, insbesondere das Fassungsvermögen des Reaktors oder Fermenters.

Der Begriff "Einbautiefe" bezieht sich auf die Position, an der ein Messsystem, wie ein Sensor, je nach Größe und Reaktorinhalt in den Biogasreaktor eingebracht wird. Die Einbautiefe soll hierbei den Überstand an festem und flüssigem Reaktorinhalt (hier vereinfacht auch als "Flüssigkeitsüberstand" bezeichnet) darstellen, der aufgrund seines hydrostatischen Drucks einen Einfluss auf die Werte des Partialdrucks des Kohlendioxids hat. Größere Einbautiefe, d.h. höherer hydrostatischer Druck des flüssigen Reaktorinhalts, führt zu einem höheren Gesamtdruck, wodurch auch die Partialdrücke der einzelnen Gase ansteigen.

Der Ausdruck "Biogaspraxis" bezeichnet die Umsetzung von Biogasanlagen in die Praxis, so dass Bedingungen im täglichen Gebrauch gemeint sind.

**Detaillierte Beschreibung der Erfindung**

Die vorliegende Erfindung stellt demnach ein Verfahren zum automatischen Überwachen der Stabilität der Methanerzeugung in einem oder mehreren Biogasreaktoren zur anaeroben Vergärung von organischer Substanz zur Verfügung, wobei der $CO_2$-Partialdruck ($pCO_2$) im flüssigen Reaktorinhalt, bei gleichzeitigem Vorhandensein des festen Reaktorinhalts, unter anaeroben Messbedingungen mit einem Messsystem während der Biogaserzeugung bestimmt wird und die automatisch in regelmäßig einstellbaren Zeitabständen ermittelten $pCO_2$-Messwerte zur prozesstechnischen Bewertung als Parameter zur Stabilitätsbeurteilung des Prozesses der Biogas- und Methanbildung herangezogen werden.

Von besonderem Vorteil beim erfindungsgemäßen Verfahren ist es dabei, dass der $CO_2$-Partialdruck ($pCO_2$) im flüssigen Reaktorinhalt alle für die Methanbildung wichtigen Prozesse, eine Substratunterversorgung oder umgekehrt eine Überlastung, die zur Übersäuerung des Biogasreaktors führen kann, integriert. Damit ist der $CO_2$-Partialdruck im Reaktorinhalt der ideale Indikator der Prozessstabilität der anaeroben biologischen Methanerzeugung. Es handelt sich daher um ein prozessbegleitendes Verfahren zur Stabilitätsbeurteilung einer Biogasanlage.

Der $CO_2$-Partialdruck kann direkt im Biogasreaktor ohne großen technischen Mehraufwand gemessen werden, so dass hierdurch ein Rückschluss auf die Prozessstabilität möglich wird und die gewonnen Informationen an den Anlagenbetreiber zeitnah weitergegeben werden können.

Es soll an dieser Stelle darauf hingewiesen werden, dass es aus dem Stand der Technik bekannt ist, die $CO_2$-Konzentration bzw. den $CO_2$-Partialdruck im Gasraum eines Reaktors zur Beurteilung eines Prozesses einzusetzen. Beispielhaft sei hierzu auf die US 4 444 882 verwiesen. Diese offenbart Verfahren zur Steuerung des Züchtens von Mikroorganismen in Kulturmedium in einem Zuchtbehälter, wobei u.a. eine Messung der Kohlendioxidkonzentration in dem ausströmenden Gas während des Züchtens durchgeführt wird. Aus der Menge des gebildeten $CO_2$ wird dann die Prozessintensität bestimmt, d.h. je mehr $CO_2$ gebildet wird, umso höher ist die Produktion. Dieses Vorgehen steht jedoch in völligem Gegensatz zur Methanerzeugung in Biogasanlagen, da hier die $CO_2$-Menge in der Reaktorflüssigkeit nicht der Bildungsrate des Zielproduktes Methan proportional ist.

Weiterhin bezieht sich die US 4 444 882 nicht auf die Methanbildung, sondern auf intermediäre Gärprodukte, insbesondere Ethanol. Im Unterschied hierzu wird bei der Methanbildung aus den Gärprodukten und insbesondere aus $CO_2$ und $H_2$ Methan durch Archaeen synthetisiert. Während bei der Kultivierung von Mikroorganismen die Bildung von $CO_2$ als Prozessparameter herangezogen wird, beruht die vorliegende Erfindung auf der Erfassung des Gleichgewichts zwischen $CO_2$ aus mikrobiellen Umsätzen und darüber hinaus der $CO_2$-Freisetzung aus dem Karbonatpuffersystem des Fermenters (bedingt durch die einhergehende Säurebelastung) auf der einen sowie dem $CO_2$-Verbrauch durch die Methanbildung auf der anderen Seite (siehe hierzu auch Figur 1).

Der Stand der Technik, welcher $CO_2$ als Prozessparameter heranzieht, berücksichtigt die Belastung des Puffersystems des Biogasfermenters durch die gebildeten Säuren regelmäßig nicht. Die gebildeten Säuren führen ebenfalls zu einer $CO_2$-Freisetzung aus dem Karbonatpuffersystem und resultieren somit nicht direkt aus der metabolischen Aktivität der beteiligten Mikroorganismen.

Darüber hinaus bezieht sich die US-Patentschrift 4 444 882 auf die $CO_2$-Produktion alleine als Parameter der Prozesseffizienz. In der vorliegenden Lehre der Erfindung geht es dagegen um die Stabilität des anaeroben Abbaus der organischen Substanz bis hin zur Methanbildung.

Eine $CO_2$-Messung in der Gasphase eines Biogasreaktors kann nicht als Maß für die Prozesseffizienz bzw. -intensität herangezogen werden, wie bereits erläutert wurde, und dies wird daher in der Biogaspraxis auch nicht angewandt.

Die $pCO_2$-Messung erfolgt in der vorliegenden Erfindung nicht in der Gasphase (headspace, Gasraum) des Reaktors, sondern direkt in der Maische (Fermentationsbrühe), womit sie sich eindeutig von anderen Veröffentlichungen

aus dem Stand der Technik unterscheidet. Die Gasphase des Reaktors besteht je nach eingesetztem Substrat und Prozessbedingungen zu 40 bis 50 % aus $CO_2$. Die Gaskonzentrationen in der Gasphase des anaeroben Biogasreaktors sind zu träge, so dass ähnliche Probleme wie beim pH-Wert vorliegen, zudem treten Verdünnungseffekte auf. Die Gasphase des Biogasreaktors ist nur eine von drei wesentlichen "Senken" für das produzierte $CO_2$ (siehe auch die Erläuterungen zu Figur 3). Daher ist die $CO_2$-Konzentration bzw. der $CO_2$-Partialdruck in der Gasphase für eine prozesstechnische Bewertung der Methanisierung völlig ungeeignet.

Der $CO_2$-Partialdruck in der Flüssigkeit eines anaeroben Biogasreaktors resultiert aus einem Zusammenwirken von verschiedenen biogenen Entstehungsprozessen und der Pufferwirkung des Hydrogenkarbonats sowie den Stoffwechselraten der hydrogenotrophen, d.h. Wasserstoffverwertenden, und $CO_2$-verbrauchenden methanogenen Archaeen. Dadurch unterscheidet sich die technische Lehre der vorliegenden Erfindung von den Lehren des Standes der Technik, welche den $CO_2$-Partialdruck in einem Reaktor proportional der Stoffwechselrate bzw. der Syntheseintensität des gewünschten Produktes setzen. Ein weiterer Unterschied besteht auch darin, dass es sich bei der vorliegenden Erfindung nicht um Prozessintensität sondern um Prozessstabilität handelt. Daher ist dieser bekannte Ansatz aus dem Stand der Technik zum $CO_2$-Partialdruck auf die biogene Methanogenese nicht übertragbar und auch nicht anwendbar.

Der $CO_2$-Partialdruck im flüssig/festen Fermenterinhalt resultiert zum Großteil aus dem mikrobiellen Abbau des zugeführten Substrates. Zusätzlich belastet die Säureproduktion während des anaeroben Abbaus der organischen Substrate das Puffersystem des Fermenters. In Biogasfermentern ist bei den erforderlichen pH-Werten zwischen 7,0 und 8,2 das Bicarbonat-Puffersystem am wichtigsten. Dabei bedingt eine Säurebelastung einen sofortigen Zerfall der Kohlensäure zu Kohlendioxid, womit der $CO_2$-Partialdruck im Gärsubstrat ansteigt. Für eine Änderung der $pCO_2$-Werte im flüssig/festen Reaktorinhalt sind somit neben den mikrobiellen Stoffwechselaktivitäten auch die Pufferreaktionen verantwortlich.

In der letzten Stufe des anaeroben Substratabbaus wird von spezialisierten Archaeen aus $H_2$ und $CO_2$ in der hydrogenotrophen Methanogenese Methan gebildet. Dieser Prozess führt im Gegensatz zu den Prozessen in den Stufen 1 bis 3 (siehe Figur 1) zu einer Absenkung des $pCO_2$ im Reaktorinhalt. In die gleiche Richtung wirkt auch der Säureabbau, d.h. die Methanbildung aus Acetat, da damit das Karbonatpuffersystem entlastet wird. Der $CO_2$-Partialdruck in der Reaktorflüssigkeit ist somit ein Resultat von gegenläufigen Quellen-Senken Prozessen.

Figur 1 zeigt in schematischer Art und Weise wie die $pCO_2$-Messung an der Schnittstelle der beschriebenen Prozesse ansetzt. Dies wird durch die beiden aufeinander zu gerichteten Pfeile jeweils rechts und links neben dem Flussdiagramm dargestellt. Auf der linken Seite von Figur 1 zeigt der obere Pfeil die $CO_2$-Produktion; der untere Pfeil stellt den $CO_2$-Verbrauch während der Biogas-Erzeugung dar. Auf der rechten Seite von Figur 1 zeigt der obere Pfeil die Belastung des Puffers durch die Säureproduktion; der zweite untere Pfeil auf der rechten Seite von Figur 1 stellt den Säure-Verbrauch dar.

Die oben geschilderten Zusammenhänge sind auch in den Figuren 2 und 3 dargestellt. So zeigt Figur 2 relativ zueinander die verschiedenen relativen Konzentrationen der anorganischen Kohlenstoffspezies, aufgetragen gegen den pH-Wert. Die nachfolgende Reaktionsgleichung stellt die für den Partialdruck des $CO_2$ relevanten Gleichgewichte im Biogasreaktor dar:

$$CO_2\,(g) \leftrightarrow CO_2\,(l) \xLeftrightarrow{+\,H_2O} H_2CO_3 \xLeftrightarrow{+\,H_2O} HCO_3^- + H_3O^+ \qquad (3)$$

Kurve (1) zeigt nun die Konzentration des Gleichgewichts aus Kohlensäure/Kohlendioxid; Kurve (2) zeigt die Konzentration von $HCO_3^-$ und Kurve (3) zeigt die Konzentration von $CO_3^{2-}$ in der flüssigen Phase im Bioreaktor, jeweils in Abhängigkeit vom pH-Wert. Die Biogasbildung erfolgt dabei in dem gezeigten pH-Wert-Bereich von etwa 7,0 bis 8,2, wobei die gegenläufig verlaufenden Kurven (1) und (2) sowie (2) und (3) die gegenläufigen Konzentrationen der unterschiedlichen Komponenten wiedergeben.

Figur 3 veranschaulicht das Quellen/Senken-Konzept des $CO_2$-Umsatzes in einem anaeroben Biogasreaktor. "Quellen" sind dabei die Erzeuger und "Senken" die Verbraucher. Dies ist ein anerkanntes Konzept, das in der Biologie, Klimaforschung, der Elektrotechnik, Logistik und in den Sozialwissenschaften (Migration) als theoretische Basis verwendet wird (s. beispielsweise Fischlin, A., Buchter, B., Matile, L., Hofer, P., Taverna, R. (2006): CO2-Senken und -Quellen in der Waldwirtschaft - Anrechnung im Rahmen des Kyoto-Protokolls, Umwelt-Wissen Nr. 0602, Bundesamt für Umwelt, Bern, 45 S.1-47 und Tian-Gen Chang and Xin-Guang Zhu (2017): Source-sink interaction: a century old concept under the light of modern molecular systems biology, Journal of Experimental Botany, Bd. 68, Nr. 16, S. 4417-4431). Figur 3 zeigt als Quellen des $CO_2$ den anaeroben Substratabbau sowie das Hydrogenkarbonat (bei pH-Abnahme). Die Senken, also Verbraucher, des $CO_2$ sind der Gasraum des Reaktors, die Methanogenese und das Hydrogenkarbonat (bei pH-Zunahme). Vernachlässigt werden hierbei der Einbau von $CO_2$ in die mikrobielle feste Biomasse sowie der Umsatz von Acetat. Die Quellen und Senken stehen einander gegenüber und bestimmen den $CO_2$-Partialdruck ($pCO_2$), der in Figur 3 als Pfeil zwischen Quelle und Senke angegeben ist. Diese Figur veranschaulicht daher,

wie die Messung des $CO_2$-Partialdrucks es ermöglicht, Informationen über die im Biogasreaktor ablaufenden Gleichgewichtsreaktionen zu erhalten.

Der Biogasreaktor, in dem das erfindungsgemäße Verfahren eingesetzt wird, kann ein- oder mehrstufig sein. Im einstufigen Prozess der Biogaserzeugung laufen die beschriebenen Abbaureaktionen in einem einzigen Reaktorsystem simultan ab. In mehrstufigen Prozessen laufen einzelne der beschriebenen Schritte in getrennten Reaktionsgefäßen ab. Bei mehrstufigen Reaktoren kann die Messung beispielweise in den jeweiligen Stufen des Reaktors erfolgen oder auf eine Stufe (insbesondere der Methanbildung) beschränkt werden. Erfindungsgemäß ist jedoch ein einstufiger Biogasreaktor bevorzugt.

Erfindungsgemäß kommen nur solche Bioreaktoren in Frage, in denen eine Nassfermentation oder Nassvergärung stattfindet. Bioreaktoren, die mit Trockenfermentation betrieben werden, werden hier nicht betrachtet. Üblicherweise findet bei der Nassfermentation ein relativ hoher Wasseranteil im Gärsubstrat Verwendung, wodurch die Masse rühr- und fließfähig wird, so dass eine Durchmischung möglich ist. Bei der Nassvergärung wird daher zumeist die feste organische Substanz oder Biomasse gut zerkleinert und mit Flüssigkeit pumpbar gehalten.

Der verwendete Bioreaktor wird vorzugsweise in kontinuierlichem Verfahren betrieben, wobei in regelmäßigen Abständen, zumeist mehrmals täglich, Substrat zugeführt wird. Das gebildete Biogas wird verwertet und das ausfermentierte Substrat (Gärrest) in nachgeschaltete Reaktoren/Behälter geführt. Es können auch mehrerer Bioreaktoren zusammengeschaltet werden. Eine Automatisierbarkeit der Anlage kann dann eine relativ gleichmäßige Gasproduktion ermöglichen.

Die Messung des $CO_2$-Partialdrucks erfolgt erfindungsgemäß direkt im Biogasreaktor und zwar im zu vergärenden Gut. Dieses zu vergärende Gut in Form von organischer Substanz setzt sich aus einer flüssigen Phase und einer festen Phase zusammen, die beide vermischt sind. Die Messung des $CO_2$-Partialdrucks erfolgt daher in der vermischten flüssigen und festen Phase.

Wenn in der vorliegenden Erfindung auf die Messung im flüssigen Reaktorinhalt, d.h. eigentlich nur in der Flüssigphase, des Bioreaktors verwiesen wird, so bedeutet dies, dass dennoch die feste Phase im Bioreaktor während der Messung vorhanden ist und nicht zur Messung entfernt wurde. Vielmehr liegen die Festphase und Flüssigphase bei der Messung des $CO_2$-Partialdrucks gleichzeitig im Bioreaktor vor; die Festphase stört die Messung nicht. Die Masse aus Feststoff und Flüssigkeit im Bioreaktor stellt daher insgesamt den "flüssigen" Reaktorinhalt dar, wobei in der Messung aber nur der Partialdruck des $CO_2$ im flüssigen Anteil des Reaktorinhalts bestimmt wird. Das $CO_2$ löst sich insbesondere in der flüssigen Phase, so dass daher und aufgrund von messtechnischen Vorgaben nur der Partialdruck in der flüssigen Phase gemessen wird. Wie es sich gezeigt hat, kann der in der organischen festen Substanz gelöste $CO_2$ tatsächlich vernachlässigt werden.

Vorteilhafterweise werden als Messsystem zur Bestimmung des $CO_2$-Partialdrucks ein oder mehrere Sensoren eingesetzt, die den Partialdruck des $CO_2$ messen. Je nach Bauart des Biogasreaktors und der Zugänge in den Reaktor kann der Sensor an beliebiger Stelle, beispielsweise von oben oder von der Seite, in den Bioreaktor eingeführt werden. Von Bedeutung in diesem Zusammenhang ist nur, dass der Sensor in die organische Substanz, d.h. feste und flüssige Phase, vollständig eintaucht und dass die anaeroben Bedingungen im Biogasreaktor erhalten bleiben. Bevorzugt und zweckmäßigerweise wird der Bioreaktorinhalt gerührt, so dass der Sensor beim regelmäßigen Rühren des Reaktorinhaltes umspült wird.

Der bevorzugt eingesetzte Sensor, insbesondere ein elektrochemischer oder ein chemoluminsezenter Sensor, nutzt zur Messung des $CO_2$-Partialdrucks in der Flüssigphase des Biogasreaktors den sauren Charakter des gelösten $CO_2$. Das $CO_2$ aus der Lösung diffundiert über eine Membran des Sensors und verändert den pH-Wert der im Sensor befindlichen Lösung. Diese pH-Wert-Änderung wird als $CO_2$-Partialdruck oder $CO_2$-Konzentration geräteintern umgerechnet und angezeigt. Die Bewertung und Interpretation der ermittelten Werte liefert Rückschlüsse auf die Prozessstabilität der im Bioreaktor ablaufenden Reaktionen.

Die Erfassung und Messung des $CO_2$-Partialdrucks direkt im anaeroben flüssigen Reaktorinhalt vermeidet die Kontamination des Reaktorinhalts durch Sauerstoff und schließt eine Veränderung des $CO_2$-Partialdrucks durch eine Probenentnahme und anschließende externe Messung der Probe aus. Die Messung des $CO_2$-Partialdrucks muss daher unmittelbar im Reaktor und zwar im Reaktorinhalt selbst erfolgen, eine Messung in einer entnommenen Probe ist erfindungsgemäß ausgeschlossen.

Als Sensor kann ein kommerziell verfügbarer $CO_2$-Sensor (z.B. Hersteller Mettler-Toledo GmbH, Presens GmbH, u.a.) verwendet werden. Die Verwendung solcher Sensoren ist bislang beispielweise aus der Biotechologie und der Biererzeugung bekannt geworden. Beispielweise wird in der WO 2014/140703 A1 die $CO_2$-Konzentration als Parameter für die Ethanolproduktion eingesetzt. Anwendungen in der Gärung mit dem Zielprodukt Ethanol unterscheiden sich jedoch deutlich von der technischen Lehre der vorliegenden Erfindung. Die Alkoholgärung hat zum Ziel, im zweiten Schritt des anaeroben Abbaus, die Bildung von Ethanol zu erzielen. In der Biogasproduktion ist Ethanol nicht bevorzugt und man geht noch zwei Stufen weiter: Bildung von Fettsäuren, Acetat, $CO_2$ und $H_2$ und deren Abbau in der Methanogenese. Das Gleichgewicht zwischen den beschriebenen Stufen des anaeroben Abbaus bei der Biogasbildung ist dabei für eine effiziente Methanproduktion entscheidend. Die bislang bekannten Anwendungen der Messung des $CO_2$-Parti-

aldrucks, wie in der Ethanolproduktion (s. WO 2014/140703 A1) sowie weiterer Stand der Technik, welcher die $CO_2$-Produktion als Parameter nutzt (s. US 4 444 882), sind - wie bereits im Einzelnen erläutert - nicht auf die Stabilität der Biogas- bzw. Methanbildung übertragbar.

Erfindungsgemäß wird bevorzugt ein automatisches $pCO_2$-Messsystem verwendet, das in regelmäßigen einstellbaren Zeitabständen den Partialdruck bestimmt. Besonders bevorzugt weist das automatische $pCO_2$-Messsystem auch eine einstellbare zeitliche Auflösung auf, so dass in einfacher Weise eine Ermittlung des $CO_2$-Partialdrucks, abhängig von der Zeit, erfolgen kann.

Eine weitere vorteilhafte Ausgestaltung der vorliegenden Erfindung ist, dass das $pCO_2$-Messsystem online verwendbar sein kann und gegebenenfalls zur automatischen Anlagensteuerung, insbesondere einer Automatisierung der Substratzufuhr zum Biogasreaktor, herangezogen werden kann.

Üblicherweise zeigen $pCO_2$-Werte unterhalb von 80 hPa eine geringe Auslastung eines Biogasreaktors an, so dass die Substratzufuhr erhöht werden und die Biogasanlage höhere Erträge liefern kann. Der Optimalbereich der $pCO_2$-Werte in einem Biogasreaktor liegt bei einer Temperatur von etwa 38°C bis 55°C beispielweise bevorzugt im Bereich von etwa 80 bis etwa 200 hPa bevorzugter im Bereich von etwa 80 bis etwa 150 hPa, noch bevorzugter im Bereich von etwa 80 bis 130 hPa. Als Untergrenze kommen beispielweise auch folgende Werte in Betracht: 85 hPa, 90 hPa, 95 hPa oder 100 hPa. Als Obergrenze kommen beispielweise auch folgende Werte in Betracht: 195 hPA, 190 hPa, 185 hPA, 180 hPa, 175 hPa, 170, hPa, 165 hPa, 160 hPa, 155 hPa, 150 hPa, 145 hPa, 140 hPa oder 135 hPa. Die Substratumsetzung ist im gewählten Bereich dann häufig optimal und es besteht keine Gefahr der Destabilisierung. Werte für den $CO_2$-Partialdruck beispielweise von etwa 200 bis etwa 400 hPa indizieren in der Regel eine Überlastung und potentielle Prozessinstabilität, die Substratzufuhr sollte dann möglichst deutlich reduziert werden. Werte beispielweise über etwa 400 hPa zeigen einen kritischen Zustand an, der ein sofortiges Einstellen der Substratzufuhr erforderlich machen würde. Dabei ist zu beachten, dass die $pCO_2$-Werte unmittelbar nach der Substrateinbringung in den Biogasreaktor zunächst ansteigen, wodurch Maximalwerte beim $CO_2$-Partialdruck resultieren, die aber nicht beim erfindungsgemäßen Verfahren zur Beurteilung der Prozessstabilität berücksichtigt werden. Diese durch Substratzufuhr hervorgerufenen Maximalwerte stellen ein kurzfristiges Ansteigen und unmittelbar danach erfolgendes Abfallen des $CO_2$-Partialdrucks dar. Für die Stabilitätsbeurteilung sind erst die Werte nach dem Abklingen dieser Maximalwerte (Peaks) für den $CO_2$-Partialdruck zu berücksichtigen, was besonders bei Biogasanlagen mit einer zeitlich flexiblen Substratzufuhr von Bedeutung ist. Es versteht sich daher, dass die durch die Substratzufuhr hervorgerufenen Maximalwerte bei den $CO_2$-Partialdruckwerte-Bereichen zur Beurteilung der Prozessstabilität gemäß der vorliegenden Erfindung außer acht gelassen werden.

Die pro Zeiteinheit gebildete Biogasmenge sowie deren Methangehalt werden durch die stoffliche Zusammensetzung der vergorenen Stoffe und ihren Aufschlusszustand, die vorhandenen Mikroorganismen, der je Zeiteinheit zugegebenen Substratmenge, der Verweilzeit im Gärbehälter sowie durch Prozessbedingungen, wie Temperatur, pH-Wert und Durchmischung, bestimmt. Über das genaue Zusammenspiel der Mikroorganismen ist hierbei nur relativ wenig bekannt. Daher ist es zweckmäßig, die verschiedenen Parameter, wie Substratart, Substratmenge, Temperatur, Einbautiefe des $pCO_2$-Sensors, Rührwerkseinstellungen etc., in jedem Einzelfall zu berücksichtigen. Beispielsweise können die Temperaturen im Biogasreaktor üblicherweise im Bereich von 38 bis 55 °C liegen. Der Druck im Biogasreaktor liegt in der Regel nur 3 bis max. 10 hPa über dem Atmosphärendruck und kann daher im Unterschied zur Einbautiefe des $pCO_2$-Sensors vernachlässigt werden.

Jedoch kann die Einbautiefe des Messsystems eine Rolle spielen. Die Einbautiefe bezeichnet hier die Tiefe in der ein Messsystem im Reaktorinhalt angeordnet ist, d.h. den Überstand an flüssig-fester Mischung in Form des Reaktorinhalts (vereinfacht als Flüssigkeitsüberstand bezeichnet), der oberhalb des Messsystems vorliegt. Mit zunehmender Einbautiefe steigt auch der $CO_2$-Partialdruck.

Die Erhöhung des $CO_2$-Partialdrucks bedingt durch die Einbautiefe bzw. den Flüssigkeitsüberstand im Hinblick auf das Messsystem kann wie folgt bestimmt werden:
Ein Meter Wassersäule entspricht einem Megapond pro Quadratmeter und damit unter Normfallbeschleunigung 9,807 kPa (rund 0,1 bar). Umgerechnet entspricht daher 1 m Tiefe im Biogasreaktor einem zusätzlichen Druck der Wassersäule von 98 hPa (siehe auch https://rechneronline.de/barometer/wasserdruck.php)

Wenn ein Biogasreaktor bei Normaldruck von 1013 hPa betrieben wird, so beträgt der Gesamtdruck am Einbauort bei einer Einbautiefe des Sensors von 1 m 1111 hPa. Wenn man nun den Partialdruck für $CO_2$ in der Fermenterflüssigkeit in 1 m Tiefe misst, erhöht sich der Gesamtdruck bei 1 m um etwa 10 %, bei 2 m Tiefe um etwa 20 % etc.

Vereinfacht kann man daher annehmen, dass sich in den oben genannten bevorzugten Bereichen die $pCO_2$-Werte je nach Einbautiefe des Messsystems für die Oberund Untergrenze pro Meter Flüssigkeitsüberstand jeweils um etwa 10 hPa erhöhen.

Wenn zum Beispiel eine Einbautiefe des Messsystems, insbesondere des Sensors, von 3 m vorliegt, dann ist das Messsystem direkt im Reaktor so angeordnet, dass sich 3 m flüssiger Reaktorinhalt oder Flüssigkeitsüberstand oberhalb des Messsystems befinden. Dann sollte bei den ausgewählten Bereichen für den $CO_2$-Partialdruck beachtet werden, dass sich die Ober- und Untergrenze des Bereichs für die $pCO_2$-Werte um jeweils $3 \times 10$ hPa erhöhen.

Dieser Zusammenhang kann anhand von Beispielen wie folgt verdeutlicht werden: Die Einbautiefe wird unmittelbar unterhalb der Flüssigkeitsoberfläche als 0 m angenommen, so dass ein Wertebereich von etwa 80 bis etwa 200 hPa ausgewählt werden kann.

Bei einer Einbautiefe des Messsystems von 3 m und einem angenommenen optimalen Wertebereich von 80 bis 200 hPa, verschiebt sich der Wertebereich für die $pCO_2$-Werte dann um jeweils 3 x 10 hPa auf (80 + 30 =) 110 hPa bis (200 + 30 =) 230 hPa; d.h. bei einer Einbautiefe von 3 m wäre der Bereich für den optimalen $CO_2$-Partialdruck dann 110 bis 230 hPa.

Bei einer Einbautiefe des Messsystems von 5 m würde sich der Wertebereich um jeweils 5 x 10 hPa auf (80 + 50 =) 130 hPa bis (200 + 50 =) 250 hPa verschieben.

Viele Maßnahmen beruhen hierbei auf Erfahrungswerten. Es versteht sich daher, dass auch der Partialdruck des $CO_2$ durch eine Vielzahl von Parametern von einem Bioreaktor zum anderen entsprechend beeinflusst und geändert werden kann. Die oben angegebenen Bereiche für den $CO_2$-Partialdruck sind daher nur als Faustregel aufzufassen, die im Einzelfall auch deutlich abweichen können. Der oben angegebene Optimalbereich der Prozessführung ist daher lediglich eine Orientierungshilfe und kann sich anlagenspezifisch und je nach Beschaffenheit der Fermenterbiologie verschieben.

Neben der Beurteilung der Prozessstabilität und Effizienz wird durch das erfindungsgemäße Verfahren daher auch eine online-Überwachung in Echtzeit möglich. Diese Echtzeit-Prozessüberwachung macht eine Automatisierung der Biogaserzeugung möglich.

Ein weiterer Vorteil der vorliegenden Erfindung ist, dass bei der online-Verwendung eines $pCO_2$-Messsystems zur Anlagesteuerung diese im Unterschied zu anderen komplexen Mess- und Regelansätzen in Biogasanlagen (vgl. DE 10 2011 110 638 A1) mit nur einem direkt im Biogasreaktor gemessenen Parameter, nämlich dem $CO_2$-Partialdruck, auskommt. Andere Parameter müssen zur Beurteilung der Prozessstabilität der im Biogasreaktor ablaufenden Reaktionen nicht herangezogen werden.

Eine vorteilhafte Ausgestaltung der Erfindung entsteht weiterhin dadurch, dass der $pCO_2$-Sensor je nach Bauart des Biogasreaktors und der Zugänge in den anaeroben Fermenter an beliebiger Stelle in den flüssigen Reaktorinhalt bzw. die Maische eingeführt werden kann, was beispielsweise durch die Reaktordecke oder seitlich durch vorhandene Öffnungen in der Reaktorwand erfolgen kann. Dieser Vorteil kommt insbesondere dadurch zum Tragen, dass hierdurch auch kleine optische Sensoren, beispielsweise mit Durchmessern im Bereich von wenigen mm (Fa. Presens GmbH: Durchmesser: 3 mm), ohne weiteres eingesetzt werden können.

Es hat sich als besonders zweckmäßig herausgestellt, wenn das Messsystem in die Reaktorflüssigkeit eingetaucht ist und beim regelmäßigen Rühren des Bioreaktorinhaltes umspült wird. Beim Einbringen des Messsystems in den Bioreaktor ist es in jedem Fall sinnvoll, wegen des hydrostatischen Drucks der Reaktorflüssigkeit die Einbautiefe zu berücksichtigen (siehe oben). Größere Einbautiefen (d.h. höherer hydrostatischer Druck der Fermenterflüssigkeit) führen zu einem höheren Gesamtdruck, wodurch auch die Partialdrücke der einzelnen Gase - wie bereits erläutert - ansteigen.

Besonders vorteilhaft an der Lehre der vorliegenden Erfindung ist, dass ohne Entnahmen aus dem Reaktorinhalt eine störungsfreie Beurteilung der Prozessstabilität unmittelbar im Biogasreaktor durch sofortige Anzeige des entsprechenden Wertes an einer Schnittstelle bzw. am PC-Monitor erfolgen kann. Die Überwachung der Prozessstabilität eines Bioreaktors kann dann beispielsweise auch durch mobile Geräte erfolgen, wodurch man in Echtzeit die aktuellen Werte des $CO_2$-Partialdrucks bekommt. Im Abgleich mit Grenzwerten, die nicht über- bzw. unterschritten werden sollen, kann daraus der Anlagenbetreiber unmittelbar entsprechende Maßnahmen ableiten.

Eine vorteilhafte Ausgestaltung der Erfindung besteht ferner darin, dass für die Umsetzung des erfindungsgemäßen Verfahrens keine zusätzlichen Ein- und Umbauten am Bioreaktor erforderlich sind, wie dies im Stand der Technik sehr häufig der Fall ist.

Ein weiterer Vorteil ist, dass das Verfahren der vorliegenden Erfindung in Bioreaktoren aller Art zum Einsatz kommen kann. Ungeachtet der Größe des Bioreaktors und des Reaktorvolumens kann das Verfahren die Stabilität der Biogaserzeugung automatisch überwachen. Das Verfahren zur Stabilitätsbeurteilung des Biogasprozesses kann daher sowohl im Labor- und Pilotmaßstab als auch im großindustriellen Maßstab erfolgen.

Im Hinblick auf die biologische Methanisierung, insbesondere die biologische Methanisierung im *in* situ-Verfahren, ist festzuhalten, dass die obigen Ausführungen hierfür gleichermaßen gelten. Die Messung des $CO_2$-Partialdrucks im Reaktor zur Optimierung der Methanisierung erfolgt prinzipiell analog, um die Stabilität des Prozesses zu beurteilen, wie in der vorliegenden Beschreibung im Einzelnen erläutert. Bei der biologischen Methanisierung wird jedoch Wasserstoff und gegebenenfalls auch Kohlendioxid dem Gärmaterial im Biogasreaktor zugesetzt. Hierbei wird der Wasserstoff durch Wasserelektrolyse erzeugt, wobei die zur Elektrolyse benötigte Energie bevorzugt mit Hilfe von erneuerbaren Energiequellen, beispielsweise Photovoltaik oder Windenergie, erzeugt wird. Das Kohlendioxid kann aus Abgasen von Industrieprozessen oder anderen Quellen stammen oder beispielsweise aus den Verbrennungsgasen des Blockheizkraftwerkes (BHKW) der betreffenden Biogasanlage gewonnen werden. Auch eine Rezirkulation des Biogases kann als $CO_2$-Quelle im Methanisierungsprozess dienen, wobei gleichzeitig die Methankonzentrationen im rezirkulierten Biogas

ansteigen (Biogas-upgrading).

Gemäß einer erfindungsgemäßen Ausführungsform wird bei der biologischen Methanisierung Wasserstoff und gegebenenfalls auch Kohlendioxid in den Reaktorinhalt des Biogasreaktors zugeführt und das Kohlendioxid und/oder zusätzliches Substrat, wie saures oder säurearmes Substrat, in Abhängigkeit von den ermittelten Werten für den $CO_2$-Partialdruck ($pCO_2$) dem flüssigen Reaktorinhalt zudosiert.

Wie bereits erläutert, ist es bevorzugt, wenn der gemessene Partialdruck des $CO_2$ im flüssigen Reaktorinhalt des Biogasreaktor in einem Optimalbereich liegt, der beispielweise im Bereich von etwa 80 bis etwa 200 hPa, bevorzugter im Bereich von etwa 80 bis etwa 150 hPa, noch bevorzugter im Bereich von etwa 80 bis etwa 130 hPa, liegen kann. Das Kohlendioxid und/oder das Substrat kann dann derart zum Reaktorinhalt zudosiert werden, dass der vorgegebene Wertebereich für den $CO_2$-Partialdruck nicht verlassen wird. Beispielweise durch Einbringen von Substrat, insbesondere saurem Substrat, wird eine Erhöhung des $pCO_2$ im Reaktorinhalt erzielt, da hierdurch eine $CO_2$-Freisetzung durch den anaeroben Abbau bzw. aus dem Bikarbonat-Puffer resultiert. Hierdurch gelingt es, die Prozessstabilität und -effizienz der Methanzeugung in der biologischen Methanisierung zu gewährleisten.

Die Steuerung der biologischen Methanisierung kann auch auf der Basis der Werte für die $CO_2$-Übersättigung der Reaktorflüssigkeit ($pCO_{2\,OS}$) erfolgen:
Zusätzlich zum $CO_2$-Partialdruck findet dann der pH-Wert des Reaktorinhaltes Berücksichtigung. Der pH-Wert kann beispielweise direkt durch einen in den Biogasreaktor eingeführten pH-Sensor gemessenen werden oder - wegen der Trägheit des pH-Wertes - einfacher in einer aus dem Reaktor entnommenen Probe. Aus dem pH-Wert kann die Berechnung des $CO_2$-Sättigungszustandes des Prozesses mittels der Henderson-Hasselbalch-Gleichung wie folgt durchgeführt werden (Millero, F.J. (1995), Thermodynamics of the carbon dioxide system in the oceans, Geochim. Cosmochim. Acta, 59, 661-677 und Weiss, R.F. (1974), Carbon dioxide in water and seawater: the solubility of a non-ideal gas, Mar. Chem. 2, 203-215):

$$pCO_2 = 10^{pKa\text{-}pH} \times p_{air} \qquad (4)$$

wobei pKa die Dissoziationskonstante für Kohlensäure (pKa = 6,32 bei 38 °C) ist und $p_{air}$ den atmosphärischen Druck des Messortes in hPa darstellt. Für die Messung unter den Bedingungen eines Biogasreaktors wird die Gleichung (4) wie folgt erweitert:

$$pCO_{2\,HH} = 10^{pKa\text{-}pH} \times (p_{air} + p_{Gasraum} + p_{Tiefe}) \qquad (5)$$

wobei $pCO_{2\,HH}$ der maximale $CO_2$-Sättigungszustand nach der erweiterten Henderson-Hasselbalch-Gleichung (5) ist, $p_{Gasraum}$ beschreibt den Druck im Gasraum des Reaktors (in der Biogaspraxis ist dies der Auslösewert der Überdrucksicherung) und $p_{Tiefe}$ ist die Druckkorrektur für die Tiefe des Einbauortes des $pCO_2$-Sensors innerhalb des Reaktors. Dies ist insbesondere dann zu berücksichtigen, wenn die Biogasreaktoren mit einem höheren Betriebsdruck arbeiten bzw. der Einbau des $pCO_2$-Sensors bei mehr als 3 m Flüssigkeitsüberstand erfolgt.

Der maximale $CO_2$-Sättigungszustand ($pCO_{2\,HH}$) nimmt bei steigenden pH-Werten der Reaktorflüssigkeit ab. Dabei verschiebt sich das Gleichgewicht zugunsten von $HCO_3^-$ und die $CO_2$-Menge in der Reaktormaische nimmt ab (siehe Gleichung (3) und (4) sowie Fig. 2). Die methanogenen Archaeen nehmen jedoch das $CO_2$ direkt aus der Flüssigkeit über Diffusion auf (siehe Agneessens et al . 2017, a.a.O.), wodurch bei steigenden pH-Werten den methanbildenden Archaeen immer weniger $CO_2$ für die hydrogenotrophe Methanogenese zur Verfügung steht.

Zusammen mit dem gemessenen $pCO_2$-Wert ($pCO_{2\,akt}$) kann aus dem maximalen $CO_2$-Sättigungszustand $pCO_{2\,HH}$ dann die $CO_2$-Übersättigung der Reaktorflüssigkeit ($pCO_{2\,OS}$) wie folgt berechnet werden:

$$pCO_{2\,OS} = pCO_{2\,akt} - pCO_{2\,HH} \qquad (6).$$

Die $CO_2$-Übersättigung kann dabei zusätzlich als Parameter zur Prozessbeurteilung herangezogen werden. Grundsätzlich bedeuten hohe $pCO_2$ os-Werte ($pCO_{2\,OS} > 3 \times pCO_{2\,HH}$) einen erheblichen Effizienzverlust des Gesamtprozesses, da $CO_2$ aus dem Reaktor entweicht und daher nicht mehr in der Methanogenese verstoffwechselt werden kann. Geringere $CO_2$-Übersättigung ($pCO_{2\,OS} < 1{,}2 \times pCO_{2\,HH}$) zeigt eine Substratunterversorgung an, da offensichtlich nicht ausreichende Mengen an organischem Kohlenstoff umgesetzt werden und wenig $CO_2$ für die hydrogenotrophe Methanproduktion verfügbar ist. Es ist daher insbesondere in der Anwendung bei der biologischen Methanisierung bevorzugt, wenn für die Werte der $CO_2$-Übersättigung der Reaktorflüssigkeit $pCO_{2\,os}$ für den Biogasreaktor folgendes

Effizienzkriterium berücksichtigt wird:

$$1{,}2 \times pCO_{2\,HH} < pCO_{2\,OS} < 3 \times pCO_{2\,HH}\,. \qquad (7).$$

Erfindungsgemäß ist es daher bevorzugt, wenn für den $pCO_{2\,os}$ die obigen Grenzwerte beachtet werden, d.h. die ermittelten Werte die Gleichung (7) erfüllen, und hierdurch die Biogasbildung in der Reaktormaische bei einer optimalen Ausnutzung des dort zur Verfügung stehenden gasförmigen $CO_2$ abläuft.

Es ist darauf hinzuweisen, dass verschiedene Parameter, wie Beschaffenheit der Reaktorflüssigkeit, Prozesstemperatur, Rührwerkseinstellungen, Art der Dosiereinrichtungen für $H_2$ und $CO_2$ etc. den oben geschilderten Zusammenhang gemäß Formel (7) beeinflussen können. Die oben angegebenen Bereiche für die $CO_2$-Übersättigung bei der *in situ* Methanisierung können im Einzelfall daher auch deutlich abweichen. Der oben angegebene Bereich ist daher eine Orientierungshilfe und kann sich anlagenspezifisch und je nach Art der Rührwerke und der Dosiereinrichtungen für $H_2$ und $CO_2$ verschieben.

Das Kohlendioxid kann dann in Abhängigkeit von den ermittelten Werten für die $CO_2$-Übersättigung der Reaktorflüssigkeit $pCO_{2\,os}$ für den Biogasreaktor zudosiert werden, wobei die oben angegebene Gleichung (7) als Stabilitätskriterium herangezogen wird. D.h. das Kohlendioxid wird derart zudosiert, dass die Gleichung (7) erfüllt ist:

$$1{,}2 \times pCO_{2\,HH} < pCO_{2\,OS} < 3 \times pCO_{2\,HH}\,. \qquad (7).$$

Bei der biologischen Methanisierung kann ein leichter $CO_2$-Überschuss vorteilhaft sein.

Von Bedeutung ist, dass die technische Lehre der vorliegenden Erfindung und ihre Vorteile gegenüber dem Stand der Technik ausschließlich für die Anwendungen unter anaeroben Bedingungen eines Biogasreaktors gelten. Das Verfahren ist nicht für Messungen in Proben des Reaktorinhaltes geeignet, die zwischenzeitlich, z.B. während der Probennahme, mit Sauerstoff in Kontakt kommen. Die in der zuvor anaeroben Reaktorflüssigkeit vorhandenen Reduktionsäquivalente reagieren dabei umgehend mit Sauerstoff und verzerren die nachfolgend ermittelten $pCO_2$-Werte völlig.

Es hat sich zudem herausgestellt, dass der $CO_2$-Partialdruck in der Tat als Parameter zur Stabilitätsbeurteilung bei der Biogaserzeugung geeignet ist. Versuche haben belegt, dass der bislang zur Prozessstabilität eingesetzte FOS/TAC-Wert, der kompliziert zu bestimmen ist, dieselben zuverlässigen Informationen zur Prozessstabilität liefert wie der $CO_2$-Partialdruck, aber der $CO_2$-Partialdruck in deutlich kürzerer Zeit und mit signifikant geringerem Mess-Aufwand bestimmbar ist. Im Gegensatz zur Messung der FOS/TAC-Werte ermöglicht die Bestimmung der $pCO_2$-Werte daher eine Echtzeit-Überwachung der Biogaserzeugung.

Das Verfahren gemäß der vorliegenden Erfindung zur prozesstechnischen Bewertung des $CO_2$-Partialdrucks im anaeroben Reaktorinhalt hat daher eine große Reihe an Vorteilen, die bislang bei der Überwachung des Biogasprozesses nicht erzielt werden konnten. Der $CO_2$-Partialdruck im Reaktorinhalt integriert alle Prozesse, die zu einer Überlastung und nachfolgender Übersäuerung des Biogasreaktors führen können und eignet sich daher als Parameter zur Stabilitätsbeurteilung der anaeroben biologischen Methanerzeugung. Ein besonderer Vorteil ist dabei insbesondere durch die online-Einsetzbarkeit des $pCO_2$-Messsystems gegeben, die zu einer Automatisierung der Anlagensteuerung im Allgemeinen und der Substratzufuhr im Besonderen verwendet werden kann.

Nachfolgend sollen einige beispielhafte Ausführungsformen der vorliegenden Erfindung anhand der Figuren 4 und 5 erläutert werden, ohne die Erfindung hierauf zu beschränken:

In Figur 4 ist eine beispielhafte Ausführungsform einer schematischen, vereinfachten Abbildung eines Bioreaktors dargestellt, in dem ein erfindungsgemäßes Messsystem zum Messen des $CO_2$-Partialdrucks im Reaktorinhalt vorliegt. Im Einzelnen zeigt Figur 4 einen Biogasreaktor 100. Typischerweise ist dies ein Rührkesselreaktor aus Stahlbeton oder Metall, der ein Reaktorvolumen von einigen Hundert bis mehreren Tausend Kubikmetern aufweisen kann. Der Bioreaktor 100 umfasst eine feste Reaktordecke 5 (auch eine flexible Ausführung der Reaktorabdeckung, die gleichzeitig als Gasspeicher dient, ist möglich), eine Reaktorwand 15 und einen Reaktorboden 25. Andere Ausführungen als die hier gezeigte sind ebenfalls möglich.

Im Bioreaktor 100 befindet sich der Reaktorinhalt 40, der die organische Substanz oder die zu vergärende Biomasse umfasst, aus der das methanreiche Biogas erzeugt wird. Der Reaktorinhalt 40 ist eine Mischung aus flüssigen und festen Bestandteilen, die unter anaeroben Bedingungen zur Erzeugung von Biogas dienen. Der Biogasreaktor wird unter Bedingungen der Nassfermentation betrieben, so dass ein hoher Wasseranteil im Gärsubstrat die Masse rühr- und fließfähig macht, so dass der Reaktorinhalt 40 durch einen Rührer 30 durchmischt werden kann.

Gemäß dem Verfahren der vorliegenden Erfindung werden Messsysteme für den $CO_2$-Partialdruck direkt in den flüssigen Reaktorinhalt 40, der die Feststoffe in aufgeschlämmtem Zustand enthält, eingebracht. Dies sind in Figur 4 zwei Sensoren 10.1 und 10.2, die den $CO_2$-Partialdruck bestimmen können. Hierbei sind beide Sensoren 10.1 und 10.2 jeweils vollständig in den Reaktorinhalt 40 eingetaucht. Der Sensor 10.1 wurde von der Decke 5 des Bioreaktors

100 in den Reaktorinhalt 40 eingebracht und Sensor 10.2 wurde durch eine Öffnung in der Seitenwand 15 des Bioreaktors 100 in den Reaktorinhalt 40 eingebracht. Andere Ausführungsvarianten sind ebenfalls möglich.

Bevorzugt werden die Sensoren 10.1 und 10.2 vom Reaktorinhalt 40 umspült, wenn gerührt wird.

Sensor 10.1 befindet sich in der gezeigten Ausführungsform unmittelbar unter der Oberfläche 45 im Reaktorinhalt. Die Einbautiefe ist daher minimal und spielt für die gemessenen $pCO_2$-Werte keine Rolle. Demgegenüber ist die Einbautiefe für den Sensor 10.2, dargestellt in Figur 4 mit dem Pfeil 48, deutlich größer, so dass hier der gemessene Partialdruck mit zusätzlich etwa 10 hPa pro Meter an Flüssigkeitsüberstand 48 zu korrigieren wäre.

Die gezeigten Sensoren zeigen eine beispielhafte Ausführung der Messung. In der Regel ist ein Sensor ausreichend. Dies hängt vom jeweiligen Einzelfall ab.

Es kann jede Art von Sensor 10.1, 10.2 Verwendung finden, solange dieser die Messung des $CO_2$-Partialdrucks oder einer anderen Größe, die in den $CO_2$-Partialdruck umgerechnet werden kann, ermöglicht.

Jeder Sensor 10.1, 10.2 ist jeweils mit einer Auswerteinheit 20.1 und 20.2 verbunden. Diese ermöglicht eine Anzeige der ermittelten Werte. Bevorzugt erfolgt die Anzeige in Echtzeit. Besonders bevorzugt umfassen die Sensoren 10.1 und 10.2 eine einstellbare zeitliche Auflösung, so dass die ermittelten $CO_2$-Partialdruckwerte in Abhängigkeit von der Zeit erfasst werden können.

Gemäß einer bevorzugten Ausführungsform sind die Sensoren 10.1 und 10.2 zur automatisierten Erfassung der Daten in der Lage, so dass die $pCO_2$-Werte automatisch bestimmt und erfasst werden. Durch Festlegen eines optimalen Bereichs für die $CO_2$-Partialdruckwerte des Bioreaktors 100, die beispielweise durch einen erfahrenen Fachmann in diesem Bereich festgelegt werden können, kann die Prozessstabilität des Bioreaktors dadurch überwacht werden, dass bei Unter- oder Überschreiten eines entsprechenden $pCO_2$-Werts der Anlagenbetreiber entsprechend gewarnt wird. Dies kann durch die Auswerteeinheit 20.1 und 20.2 in Echtzeit erfolgen, so dass eine Destabilisierung der Reaktionen des Bioreaktors 100 verhindert werden kann. Der Anlagenbetreiber kann dann beispielsweise durch Änderungen der Substratzufuhr entsprechend reagieren.

Ein beispielhafter Bereich für die $CO_2$-Partialdruckwerte eines Bioreaktors, der bei einer Temperatur von etwa 38 bis 55°C betrieben wird, kann im Bereich von etwa 80 bis etwa 200 hPa, bevorzugter im Bereich von etwa 80 bis etwa 150 hPa, noch bevorzugter im Bereich von etwa 80 bis etwa 130 hPa, liegen. Andere Wertebereiche je nach Bauart des Bioreaktors, zu vergärender Substanz, Temperatur, Einbautiefe des Messsystems etc. sind möglich.

Wird der Wertebereich verlassen, kann der Anlagenbetreiber mit einem Warnton, einer farblichen Anzeige oder in anderer Weise darauf aufmerksam gemacht werden. Dies hängt von der Ausgestaltung der Auswerteeinheit und der Art der weiteren Datenverarbeitung und -Übermittlung ab.

Weiterhin zeigt Figur 5 eine beispielhafte Ausführungsform der vorliegenden Erfindung einer schematischen, vereinfachten Abbildung eines Bioreaktors, der zur biologischen Methanisierung *in situ* eingesetzt wird, wobei ein erfindungsgemäßes Messsystem zum Messen des $CO_2$-Partialdrucks in der Reaktorflüssigkeit vorliegt. Im Einzelnen zeigt Figur 5 einen Biogasreaktor 200 der zur biologischen Methanisierung verwendet wird. Typischerweise ist dies ein Rührkesselreaktor aus Stahlbeton oder Metall, der ein Reaktorvolumen von einigen Hundert bis mehreren Tausend Kubikmetern aufweisen kann. Der Bioreaktor 200 umfasst eine feste Reaktordecke 5 (auch eine flexible Ausführung der Reaktorabdeckung ist möglich), eine Reaktorwand 15 und einen Reaktorboden 25. Andere Ausführungen als die hier gezeigte sind ebenfalls möglich.

Im Bioreaktor 200 befindet sich der Reaktorinhalt 40, der die organische Substanz oder zu vergärende Biomasse umfasst, aus der das methanreiche Biogas, hier vornehmlich Methan, durch biologischen Methanisierung erzeugt wird. Der Reaktorinhalt 40 ist eine Mischung aus flüssigen und festen Bestandteilen, die unter anaeroben Bedingungen zur Erzeugung von methanreichem Biogas dienen. Der Biogasreaktor 200 wird unter Bedingungen der Nassfermentation betrieben, so dass ein hoher Wasseranteil im Gärsubstrat die Masse rühr- und fließfähig macht, so dass der Reaktorinhalt 40 durch einen Rührer 30 durchmischt werden kann.

Im vorliegenden Fall liegt eine biologische Methanisierung im *in situ*-Verfahren vor, d.h. Wasserstoff für die biologische Methanisierung wird direkt in das Gärmaterial des Fermentationsprozesses gegeben. Wie in Figur 5 gezeigt, wird Wasserstoff ($H_2$) durch die Zudosierung 50 direkt in den Reaktorinhalt 40 geleitet. Im gezeigten Ausführungsbeispiel wird zudem Kohlendioxid ($CO_2$) durch die Zudosierung 60 direkt in den Reaktorinhalt 40 geleitet. Der Wasserstoff wird in der Regel aus einer Wasserelektrolyse gewonnen. Die Energie für die Wasserelektrolyse stammt bevorzugt aus erneuerbaren Energien, wie Photovoltaik oder Windenergie. Das Kohlendioxid stammt beispielweise aus Industrieabgas oder vorzugsweise aus BHKW-Abgasen der Biogasanlage.

Gemäß dem Verfahren der vorliegenden Erfindung wird das Messsystem für den $CO_2$-Partialdruck direkt in den flüssigen Reaktorinhalt 40 eingebracht. Dies sind in Figur 5 zwei Sensoren 10.1 und 10.2, die den $CO_2$-Partialdruck bestimmen. Ausgehend von den ermittelten Werten für den $CO_2$-Partialdruck kann dann das Kohlendioxid und/oder Substrat, wie saures oder säurearmes Substrat, zum flüssigen Reaktorinhalt 40 zudosiert werden.

Hierbei sind beide Sensoren 10.1 und 10.2 jeweils vollständig in den Reaktorinhalt 40 eingetaucht. Der Sensor 10.1 wurde von der Decke 5 des Bioreaktors 200 in den Reaktorinhalt 40 eingebracht und Sensor 10.2 wurde durch eine Öffnung in der Seitenwand 15 des Bioreaktors 200 in den Reaktorinhalt 40 eingebracht. Andere Ausführungsvarianten sind ebenfalls möglich.

Bevorzugt werden die Sensoren 10.1 und 10.2 vom Reaktorinhalt 40 umspült, wenn gerührt wird.

Sensor 10.1 befindet sich in der gezeigten Ausführungsform unmittelbar unter der Oberfläche 45 im Reaktorinhalt. Die Einbautiefe ist daher minimal und spielt für die gemessenen $pCO_2$-Werte keine Rolle. Demgegenüber ist die Einbautiefe für den Sensor 10.2, dargestellt in Figur 5 mit dem Pfeil 48, deutlich größer, so dass hier der gemessene Partialdruck mit zusätzlich etwa 10 hPa pro Meter an Flüssigkeitsüberstand 48 zu korrigieren wäre.

Die Zahl der gezeigten Sensoren ist beispielhaft. Es kann auch nur ein Sensor oder es können auch mehr als zwei Sensoren vorliegen, um die $CO_2$-Verfügbarkeit für die methanbildenden Archaeen räumlich genau abzubilden. Dies hängt vom jeweiligen Einzelfall ab.

Es kann jede Art von Sensor 10.1, 10.2 Verwendung finden, solange dieser die Messung des $CO_2$-Partialdrucks oder einer anderen Größe, die in den $CO_2$-Partialdruck umgerechnet werden kann, ermöglicht.

Jeder Sensor 10.1, 10.2 ist jeweils mit einer Auswerteinheit 20.1 und 20.2 verbunden. Diese ermöglicht eine Anzeige der ermittelten Werte. Bevorzugt erfolgt die Anzeige in Echtzeit. Besonders bevorzugt umfassen die Sensoren 10.1 und 10.2 eine einstellbare zeitliche Auflösung, so dass die ermittelten $CO_2$-Partialdruckwerte abhängig von der Zeit erfasst werden können.

Gemäß einer bevorzugten Ausführungsform sind die Sensoren 10.1 und 10.2 zur automatisierten Erfassung der Daten in der Lage, so dass die $pCO_2$-Werte automatisch bestimmt und erfasst werden. Durch Festlegen eines optimalen Bereichs für die $CO_2$-Partialdruckwerte des Bioreaktors 200, die beispielweise durch einen erfahrenen Fachmann in diesem Bereich festgelegt werden können, kann die Prozessstabilität und -effizienz des Bioreaktors dadurch überwacht werden, dass bei Unter- oder Überschreiten eines entsprechenden $pCO_2$-Werte der Anlagenbetreiber entsprechend gewarnt wird. Dies kann durch die Auswerteeinheit 20.1 und 20.2 in Echtzeit erfolgen, so dass eine Destabilisierung der Reaktionen des Bioreaktors 200 verhindert werden kann. Der Anlagenbetreiber kann dann beispielsweise durch Erhöhung der $CO_2$-Zufuhr oder Reduzierung der $CO_2$-Zufuhr und/oder Substratzufuhr-Erhöhung oder -Reduktion entsprechend reagieren.

Ein beispielhafter Bereich für die $CO_2$-Partialdruckwerte eines Bioreaktors, der bei einer Temperatur von etwa 40°C betrieben wird, kann von etwa 80 bis etwa 200 hPa, bevorzugter im Bereich von etwa 80 bis etwa 150 hPa, noch bevorzugter im Bereich von etwa 80 bis etwa 130 hPa, liegen. Andere Wertebereiche je nach Bauart des Bioreaktors, zu vergärender Substanz, Temperatur, Einbautiefe des Messsystems etc. sind möglich. Das Kohlendioxid kann dann so zum flüssigen (s. Anmerkung oben) Reaktorinhalt 40 zudosiert werden, dass der angegebene Wertebereich nicht verlassen wird. Der $CO_2$-Partialdruck könnte auch durch die $H_2$-Zufuhr 50 entsprechend beeinflusst werden. Dies ist jedoch ein sehr komplexes Verfahren und im Verein mit einer Steuerung der $CO_2$-Verfügbarketi über den $CO_2$ Partialdruck in der Fermenterflüssigkeit erfindungsgemäß nicht erwünscht.

Wird der vordefinierte Wertebereich verlassen, kann der Anlagenbetreiber mit einem Warnton, einer farblichen Anzeige oder in anderer Weise darauf aufmerksam gemacht werden. Dies hängt von der Ausgestaltung der Auswerteeinheit und der Art der weiteren Datenverarbeitung und -Übermittlung ab.

Gemäß einer weiteren bevorzugten Ausführungsform der Erfindung kann die Prozessstabilität in Figur 5 auch auf Basis der Werte für die $CO_2$-Übersättigung $pCO_{2\ OS}$, ausgehend von den ermittelten Werten des $CO_2$-Partialdrucks ($pCO_2$) überwacht werden:

Mit weiteren Sensoren (nicht gezeigt) kann zusätzlich der pH-Wert des flüssigen Reaktorinhaltes 40 gemessen werden, um hieraus den maximalen $CO_2$-Sättigungszustand $pCO_{2\ HH}$ zu errechnen und aus diesem, zusammen mit den ermittelten Werten für den $CO_2$-Partialdruck, den $CO_2$-Sättigungszustand $pCO_{2\ OS}$ des Prozesses zu ermitteln. Dieser kann dann im Rahmen der Gleichung (7)

$$1{,}2 \times pCO_{2\ HH} < pCO_{2\ OS} < 3 \times pCO_{2\ HH} \qquad (7)$$

als Kriterium für die biologische Methanisierung herangezogen werden, und das Kohlendioxid und/oder Substrat können ausgehend von den ermittelten Werten des $pCO_{2\ OS}$ zum Reaktorinhalt 40 zudosiert werden, damit es zu keiner $CO_2$-Unterversorgung der gleichzeitig $H_2$- und $CO_2$-verarbeitenden hydrogenotrophen, methanbildenden Archaeen kommt.

Die Erfindung wird nachfolgend anhand von Versuchen erläutert, welche die Erfindung nicht beschränken sollen.

## Versuche

Es wurden einige Versuche durchgeführt, um die vorliegende Erfindung zu veranschaulichen:

In Figur 6 wird der Zusammenhang zwischen den $CO_2$-Partialdruck-Werten und der Zufuhr von Substrat einerseits und der Reduktion der Substratgaben andererseits, durchgeführt in einer Praxis-Biogasanlage im Laufe einer Woche, veranschaulicht. Hierfür sind die ermittelten Werte für den $CO_2$-Partialdruck ($pCO_2$ in [hPa]) gegen mehrere Tage aufgetragen.

Im Einzelnen ist die $pCO_2$-Überwachung einer landwirtschaftlichen Anlage mit flexibler Substratzufuhr gezeigt. Die Anlage wurde mit Mais- sowie Ganzpflanzensilage und Schweinegülle betrieben und verfügte über eine installierte elektrische Leistung von 250 kW (250 $kW_{el}$-Anlage). Die Anlage wurde während der Messperiode mit abnehmenden Mengen an Maissilage beschickt. An Tag 1 und Tag 6 wurde jeweils die fünffache Menge an Gülle zugeführt. Die Silage wurde kontinuierlich zugeführt, mit sinkenden Mengen zum Ende der Woche. In Figur 6 sind die Ausschläge (Peaks) der $pCO_2$-Werte durch die Zufuhr der Gülle ebenso deutlich zu erkennen wie die Abnahme des $pCO_2$ durch die Reduktion der Substratmenge im Laufe der Woche.

Die Figur belegt den direkten Zusammenhang zwischen zugeführter Substratmenge und dem $CO_2$-Partialdruck.

In Figur 7 wird der Zusammenhang zwischen den $CO_2$-Partialdruck-Werten und der Zufuhr von Substrat in einer Biogasanlage in einem Langzeittest veranschaulicht.

Die Reststoff-Biogasanlage wurde mit Speiseresten, Mais- und Grassilage betrieben und verfügte über eine installierte elektrische Leistung von 350 kW. Bei kontinuierlicher Fahrweise zeigte die Anlage $pCO_2$-Werte von etwa 100 hPa. Zu Testzwecken wurden der Reststoff-Biogasanlage periodisch hohe Mengen an Speiseresten zugeführt. Danach schlugen die $pCO_2$-Werte auf bis zu 350 hPa aus und zeigten die Belastung der Fermenterbiologie unmittelbar an. Danach sanken die $pCO_2$-Werte wieder in einen unkritischen Bereich unterhalb von 100 hPa.

Figur 8 zeigt eine Biogasanlage mit akuter Prozessstörung, dargestellt anhand der FOS/TAC-Werte, der Menge der Gesamtsäuren (insbesondere Essig- und Propionsäure) sowie der $CO_2$-Partialdruck-Werte im Reaktorinhalt.

Diese Biogasanlage wurde einen Monat hinsichtlich der pCO2- und FOS/TAC-Werte sowie zwei Wochen hinsichtlich der Gesamtsäuren überwacht, wobei der Prozess durch eine hohe Säurebelastung offensichtlich gestört war. Die Anlage wird mit Mais-, Gras- und Ganzpflanzensilage betrieben und verfügte über eine installierte elektrische Leistung von 1050 kW. Zu Beginn der Messungen wurden hohe FOS/TAC-Werte von über 1,2, hohe $pCO_2$-Werte von über 400 hPa und auch hohe Gesamtsäure-Werte von über 6000 mg/l gemessen. Nachdem Maßnahmen zur Reduktion der Säurelast ergriffen wurden (wie Reduktion der Substratzufuhr, Einbringung vom aktiven säurearmen Gärrest aus einer anderen Anlage etc.) stabilisierten sich die Prozesskennzahlen. Auch die Biogas- und Methanproduktion normalisierten sich. Die Messwerte zeigten, wie sich der Prozess der Anlage im Laufe des überwachten Monats stabilisierte. Dabei zeigt sich eine deutliche Korrelation zwischen den $pCO_2$-Werten, den FOS/TAC-Werten einerseits und den Gesamtsäuren andererseits.

Figur 9 veranschaulicht den Zusammenhang zwischen den in einer Labor-Biogasanlage periodisch ermittelten FOS/TAC-Werten und dem kontinuierlich gemessenen $CO_2$-Partialdruck im Reaktorinhalt. Dieser Langzeitversuch lief über 51 Tage und zeigt, dass eine Korrelation zwischen den ermittelten FOS/TAC-Werten und dem kontinuierlich gemessenen $CO_2$-Partialdruck vorliegt. Die vorgestellten Versuche belegen damit, dass der $CO_2$-Partialdruck ein geeigneter Parameter ist, um die Prozessstabilität der Biogaserzeugung in zuverlässiger Weise zu ermöglichen.

Bezugszeichenliste

| | |
|---|---|
| 5 | Reaktordecke |
| 10.1, 10.2 | Sensor |
| 20.1, 20.2 | Auswerteeinheit |
| 15 | Seitenwand des Bioreaktors |
| 25 | Boden des Bioreaktor |
| 30 | Rührer |
| 40 | Reaktorinhalt |
| 45 | Oberfläche des Reaktorinhalts |
| 48 | Einbautiefe / Flüssigkeitsüberstand |
| 50 | $H_2$-Zudosierung |
| 60 | $CO_2$-Zudosierung |
| 100, 200 | Biogasreaktor |

**Patentansprüche**

1. Verfahren zum automatischen Überwachen der Stabilität der Methanerzeugung in einem oder mehreren Biogasreaktoren (100, 200) zur anaeroben Vergärung von organischer Substanz mit den Schritten:

   - Bestimmen des $CO_2$-Partialdrucks ($pCO_2$) im flüssigen Reaktorinhalt (40) unter anaeroben Messbedingungen mit einem Messsystem (10.1, 10.2) während der Biogaserzeugung, wobei die $pCO_2$-Messwerte automatisch

in regelmäßigen einstellbaren Zeitabständen ermittelt werden, und
- Einsetzen der ermittelten Werte zur prozesstechnischen Bewertung als Parameter zur Stabilitätsbeurteilung des Prozesses der Biogas- und Methanbildung.

**2.** Verfahren nach Anspruch 1
**dadurch gekennzeichnet,**
**dass** als Messsystem ein oder mehrere Sensoren (10.1, 10.2) eingesetzt werden, die den $CO_2$-Partialdruck messen, wobei jeder Sensor (10.1, 10.2) vollständig in den flüssigen Reaktorinhalt (40) eingetaucht und bevorzugt umspült wird, wenn der Reaktorinhalt (40) gerührt wird.

**3.** Verfahren nach einem der vorangehenden Ansprüche 1 oder 2,
**dadurch gekennzeichnet,**
**dass** das verwendete Messsystem (10.1, 10.2) ein automatisiertes Messsystem darstellt, das in regelmäßigen einstellbaren Zeitabständen den $CO_2$-Partialdruck bestimmt, und zu einer Automatisierung der Anlagensteuerung, insbesondere einer Automatisierung der Substratzufuhr zum Biogasreaktor (100), verwendet wird.

**4.** Verfahren nach Anspruch 1 bis 3,
**dadurch gekennzeichnet,**
**dass** das verwendete Messsystem (10.1, 10.2) zur online-Verwendung, insbesondere zur online-Überwachung eines Bioreaktors (100, 200) in Echtzeit, geeignet ist und bevorzugt eine einstellbare zeitliche Auflösung aufweist.

**5.** Verfahren nach einem der vorangehenden Ansprüche 1 bis 4,
**dadurch gekennzeichnet,**
**dass** das verwendete Messsystem (10.1, 10.2) durch die Reaktordecke (5) oder seitlich durch vorhandene Öffnungen in der Reaktorwand (15) des Bioreaktors (100, 200) in den flüssigen Reaktorinhalt (40) eingebracht wird.

**6.** Verfahren nach einem der vorangehenden Ansprüche 1 bis 5,
**dadurch gekennzeichnet,**
**dass** ein optimaler Bereich für die $CO_2$-Partialdruckwerte eines Bioreaktors (100), insbesondere abhängig vom Reaktorvolumen, der Temperatur, der Einbautiefe des Messsystems, der zu verarbeitenden organischen Substanz, ausgewählt wird, so dass beim Unterschreiten des gewählten Bereichs, die Substratzufuhr zum Bioreaktor (100) erhöht und beim Überschreiten des Bereichs die Substratzufuhr reduziert wird.

**7.** Verfahren nach einem der vorangehenden Ansprüche 1 bis 6,
**dadurch gekennzeichnet,**

**dass** ein optimaler Bereich für die $CO_2$-Partialdruckwerte eines Bioreaktors (100, 200), der bei einer Temperatur von etwa 38°C bis 55°C betrieben wird, im Bereich von etwa 80 bis etwa 200 hPa, bevorzugt im Bereich von etwa 80 bis etwa 150 hPa, noch bevorzugter im Bereich von etwa 80 bis etwa 130 hPa, liegt,
wobei je nach Einbautiefe des Messsystems (10.2), das den $CO_2$-Partialdruck misst, die Ober- und Untergrenze des Bereichs pro Meter Flüssigkeitsüberstand (48) jeweils um etwa 10 hPa erhöht wird.

**8.** Verfahren nach einem der vorangehenden Ansprüche 1 bis 7,
**dadurch gekennzeichnet,**
**dass** der oder die Biogasreaktoren (100, 200) kontinuierlich betrieben werden, wobei in regelmäßigen Abständen eine Substratzufuhr und Abfuhr von vergorenem Substrat durchgeführt wird.

**9.** Verfahren nach einem der vorangehenden Ansprüche 1 bis 8,
**dadurch gekennzeichnet,**
**dass** das verwendete Messsystem (10.1, 10.2) zum Bestimmen des $CO_2$-Partialdrucks im flüssigen Reaktorinhalt (40) des Biogasreaktors (100, 200) mit einer Auswerteeinheit (20.1, 20.2) verbunden ist, die die Messwerte des $CO_2$-Partialdrucks ausgibt oder die gemessenen Werte in den $CO_2$-Partialdruck umrechnet.

**10.** Verfahren nach einem der vorangehenden Ansprüche 1 bis 9,
**dadurch gekennzeichnet,**
**dass** die Methanerzeugung eine biologische Methanisierung, insbesondere eine biologische Methanisierung im *in* situ-Verfahren in einem Power-to-Gas-Verfahren, darstellt, bei der der organischen Substanz im anaeroben Reaktor (200) zusätzlich Wasserstoff (59) und gegebenenfalls Kohlendioxid (60) zugeführt werden.

**11.** Verfahren nach Anspruch 10,
**dadurch gekennzeichnet,**
**dass** Kohlendioxid und/oder Substrat, insbesondere saures oder säurearmes Substrat, in Abhängigkeit von den ermittelten Werten des $CO_2$-Partialdrucks ($pCO_2$) dem flüssigen Reaktorinhalt (40) zudosiert werden

**12.** Verfahren nach Anspruch 11,
**dadurch gekennzeichnet,**
**dass** ein optimaler Bereich für die $CO_2$-Partialdruckwerte eines Bioreaktors (200), insbesondere abhängig vom Reaktorvolumen, der Temperatur, der Einbautiefe des Messsystems (10.1, 10.2), der zu verarbeitenden organischen Substanz, ausgewählt wird, so dass bei Unterschreiten der gewählten optimalen $pCO_2$-Werte, die $CO_2$-Zufuhr (60) und/oder Substratzufuhr zum Bioreaktor (200) erhöht und bei Überschreiten der gewählten optimalen $pCO_2$-Werte die $CO_2$-Zufuhr (60) und/oder Substratzufuhr zum Bioreaktor (200) verringert wird.

**13.** Verfahren nach einem der Ansprüche 10 bis 12,
**dadurch gekennzeichnet,**
**dass** als weiteres Kriterium für die Prozessstabilität die $CO_2$-Übersättigung des flüssigen Reaktorinhalts (40) berücksichtigt wird, wobei der pH-Wert des Reaktorinhaltes (40) gemessen wird, aus dem pH-Wert der maximale $CO_2$-Sättigungszustand $pCO_{2\ HH}$ wie folgt berechnet wird:

$$pCO_{2\ HH} = 10^{pKa\text{-}pH} \times (p_{air} + p_{Gasraum} + p_{Tiefe})$$

wobei

$pCO_{2\ HH}$ den maximalen $CO_2$-Sättigungszustand nach der erweiterten Henderson-Hasselbalch-Gleichung darstellt,
$p_{Gasraum}$ den Druck im Gasraum des Reaktors (200) darstellt und
$P_{Tiefe}$ die Druckkorrektur für die Tiefe des Einbauortes (48) des $pCO_2$-Messsystems (10.2) innerhalb des Reaktors (200) darstellt;
anhand des maximalen $CO_2$-Sättigungszustands $pCO_{2\ HH}$ zusammen mit dem gemessenen $pCO_2$-Wert die $CO_2$-Übersättigung des flüssigen Reaktorinhalts (40) $pCO_{2\ os}$ wie folgt berechnet wird:

$$pCO_{2\ OS} = pCO_{2\ akt} - pCO_{2\ HH}$$

wobei
$pCO_{2\ os}$ die $CO_2$-Übersättigung des flüssigen Reaktorinhalts (40) darstellt,
$pCO_{2\ HH}$ den maximalen $CO_2$-Sättigungszustand darstellt,
$pCO_{2\ akt}$ den gemessenen $pCO_2$-Wert darstellt;
und die $CO_2$-Partialdruck-Werte im flüssigen Reaktorinhalt (40) des Bioreaktors (200) durch entsprechendes Zudosieren von Kohlendioxid (60) und/oder Substrat derart eingestellt werden, dass gilt:

$$1{,}2 \times pCO_{2\ HH} < pCO_{2\ OS} < 3 \times pCO_{2\ HH}.$$

## Claims

**1.** Method for automatic monitoring the stability of methane production in one or more biogas reactors (100, 200) for anaerobic fermentation of organic substances, comprising the steps of:

- determining the $CO_2$ partial pressure ($pCO_2$) in the liquid reactor contents (40) under anaerobic measuring conditions using a measuring system (10.1, 10.2) during biogas production, wherein the $pCO_2$ measured values are automatically determined at regular adjustable time intervals, and
- using the determined values for process-related evaluation as parameters for stability assessment of the process of biogas and methane formation.

2. Method according to claim 1,
**characterized in that**
one or more sensors (10.1, 10.2) which measure the $CO_2$ partial pressure are used as the measuring system, wherein each sensor (10.1, 10.2) is completely immersed in the liquid reactor contents (40) and is preferably flushed around when the reactor contents (40) are stirred.

3. Method according to one of the preceding claims 1 or 2,
**characterized in that**
the measuring system (10.1, 10.2) used represents an automated measuring system which determines the $CO_2$ partial pressure at regular adjustable time intervals and is used for automation of the plant control, in particular automation of the substrate feed to the biogas reactor (100).

4. Method according to claim 1 to 3,
**characterized in that**
the measuring system (10.1, 10.2) used is suitable for online use, in particular for online monitoring of a bioreactor (100, 200) in real time, and preferably has an adjustable temporal resolution.

5. Method according to one of the preceding claims 1 to 4,
**characterized in that**
the measuring system (10.1, 10.2) used is introduced into the liquid reactor contents (40) through the reactor ceiling (5) or laterally through existing openings in the reactor wall (15) of the bioreactor (100, 200).

6. Method according to one of the preceding claims 1 to 5,
**characterized in that**
an optimal range for the $CO_2$ partial pressure values of a bioreactor (100) is selected, in particular depending on the reactor volume, the temperature, the installation depth of the measuring system, the processed organic substances or material, so that when falling below the selected range, the substrate supply to the bioreactor (100) is increased and when exceeding the range, the substrate supply is reduced.

7. Method according to one of the preceding claims 1 to 6,
**characterized in that**

   an optimal range for the $CO_2$ partial pressure values of a bioreactor (100, 200) operated at a temperature of about 38°C to 55°C is in the range of about 80 to about 200 hPa, preferably in the range of about 80 to about 150 hPa, more preferably in the range of about 80 to about 130 hPa,
   wherein, depending on the installation depth of the measuring system (10.2) which measures the $CO_2$ partial pressure, the upper and lower limits of the range per meter of liquid supernatant (48) are each increased by about 10 hPa.

8. Method according to one of the preceding claims 1 to 7,
**characterized in that**
the biogas reactor or reactors (100, 200) are operated continuously, wherein substrate supply and removal of fermented substrate is carried out at regular intervals.

9. Method according to one of the preceding claims 1 to 8,
**characterized in that**
the measuring system (10.1, 10.2) used to determine the $CO_2$ partial pressure in the liquid reactor contents (40) of the biogas reactor (100, 200) is connected to an evaluation unit (20.1, 20.2) which outputs the measured values of the $CO_2$ partial pressure or converts the measured values into the $CO_2$ partial pressure.

10. Method according to one of the preceding claims 1 to 9,
**characterized in that**
the methane generation is a biological methanation, in particular a biological methanation in an *in situ* process in a power-to-gas process, in which hydrogen (59) and, optionally, carbon dioxide (60) are additionally fed to the organic substances in the anaerobic reactor (200).

11. Method according to claim 10,
**characterized in that**

carbon dioxide and/or substrate, in particular acidic or low-acid substrate, are metered into the liquid reactor contents (40) as a function of the determined values of the $CO_2$ partial pressure ($pCO_2$).

**12.** Method according to claim 11,
**characterized in that**
an optimum range for the $CO_2$ partial pressure values of a bioreactor (200) is selected, in particular as a function of the reactor volume, the temperature, the installation depth of the measuring system (10.1, 10.2), the organic substances to be processed, so that if the selected optimum $pCO_2$ values are not reached, the $CO_2$ supply (60) and/or substrate supply to the bioreactor (200) is increased and if the selected optimum $pCO_2$ values are exceeded, the $CO_2$ supply (60) and/or substrate supply to the bioreactor (200) is reduced.

**13.** Method according to one of claims 10 to 12,
**characterized in that**

the $CO_2$ supersaturation of the liquid reactor contents (40) is taken into account as a further criterion for the process stability, wherein the pH value of the reactor contents (40) is measured, from the pH value the maximum $CO_2$ saturation state $pCO_{2\,HH}$ is calculated as follows:

$$pCO_{2\,HH} = 10^{pKa-pH} \times (p_{air} + p_{gas\,space} + p_{depth})$$

wherein
$pCO_{2\,HH}$ represents the maximum $CO_2$ saturation state according to the extended Henderson-Hasselbalch equation,
$p_{gas\,space}$ represents the pressure in the gas space of the reactor (200) and
$p_{depth}$ represents the pressure correction for the depth of the installation location (48) of the $pCO_2$ measuring system (10.2) within the reactor (200);
based on the maximum $CO_2$ saturation state $pCO_{2\,HH}$ together with the measured $pCO_2$ value, the $CO_2$ supersaturation of the liquid reactor contents (40) $pCO_2$ os is calculated as follows:

$$pCO_{2\,OS} = pCO_{2\,act} - pCO_{2\,HH}$$

wherein
$pCO_{2\,OS}$ represents the $CO_2$ supersaturation of the liquid reactor contents (40),
$pCO_{2\,HH}$ represents the maximum $CO_2$ saturation state,
$pCO_{2\,act}$ represents the measured $pCO_2$ value;
and the $CO_2$ partial pressure values in the liquid reactor contents (40) of the bioreactor (200) are adjusted by appropriate metering of carbon dioxide (60) and/or substrate such that the following applies:

$$1.2 \times pCO_{2\,HH} < pCO_{2\,OS} < 3 \times pCO_{2\,HH}.$$

## Revendications

**1.** Procédé pour la surveillance automatique de la stabilité de la génération de méthane dans un ou plusieurs réacteurs à biogaz (100, 200) pour la fermentation anaérobie de substances organique, comprenant les étapes de :

- détermination de la pression partielle de $CO_2$ ($pCO_2$) dans le contenu liquide du réacteur (40) dans des conditions de mesure anaérobies avec un système de mesure (10.1, 10.2) pendant la génération de biogaz, les valeurs de mesure de la $pCO_2$ étant automatiquement déterminées à des intervalles réguliers paramétrables, et
- utilisation des valeurs déterminées comme paramètre pour l'évaluation technique de la stabilité du processus de formation de biogaz et de méthane.

**2.** Procédé selon la revendication 1, **caractérisé en ce que** le système de mesure utilisé est formé d'un ou plusieurs

capteurs (10.1, 10.2) qui mesurent la pression partielle de $CO_2$, chaque capteur (10.1, 10.2) étant complètement immergé dans le contenu liquide du réacteur (40) et de préférence parcouru par le liquide quand le contenu du réacteur (40) est remué.

**3.** Procédé selon l'une des revendications 1 ou 2, **caractérisé en ce que** le système de mesure (10.1, 10.2) utilisé représente un système de mesure automatisé qui détermine à intervalles réguliers paramétrables dans le temps la pression partielle de $CO_2$ et qui est utilisé pour automatiser la conduite de l'installation, en particulier pour automatiser l'apport de substrat au réacteur à biogaz (100).

**4.** Procédé selon les revendications 1 à 3, **caractérisé en ce que** le système de mesure (10.1, 10.2) utilisé convient pour l'utilisation en ligne, en particulier pour la surveillance en ligne d'un bioréacteur (100, 200) en temps réel, et présente de préférence une résolution dans le temps paramétrable.

**5.** Procédé selon l'une des revendications 1 à 4, **caractérisé en ce que** le système de mesure (10.1, 10.2) utilisé est introduit dans le contenu liquide du réacteur (40) à travers le couvercle du réacteur (5) ou latéralement à travers des ouvertures existantes dans la paroi de réacteur (15) du bioréacteur (100, 200).

**6.** Procédé selon l'une des revendications 1 à 5, **caractérisé en ce qu'**une plage optimale pour les valeurs de pression partielle de $CO_2$ d'un bioréacteur (100) est choisie, en particulier en fonction du volume du réacteur, de la température, de la profondeur d'installation du système de mesure, des substances organique à transformer, de sorte que si une limite inférieure de la plage choisie n'est pas atteinte, l'apport de substrat au bioréacteur (100) est augmentée et, si la plage choisie est dépassée, l'apport de substrat est réduit.

**7.** Procédé selon l'une des revendications 1 à 6, **caractérisé en ce qu'**une plage optimale des valeurs de pression partielle de $CO_2$ d'un bioréacteur (100, 200) conduit à une température d'environ 38 °C à 55 °C se situe dans une plage d'environ 80 à environ 200 hPa, de préférence dans une plage d'environ 80 à environ 150 hPa, en particulier dans une plage d'environ 80 à environ 130 hPa, les limites supérieure et inférieure de la plage étant augmentées, selon la profondeur d'installation du système de mesure (10.2) qui mesure la pression partielle de $CO_2$, d'environ 10 hPa par mètre de hauteur de liquide (48) au-dessus.

**8.** Procédé selon l'une des revendications 1 à 7, **caractérisé en ce que** le ou les réacteurs à biogaz (100, 200) sont conduits en continu, avec un apport de substrat et une évacuation de substrat fermenté à intervalles réguliers.

**9.** Procédé selon l'une des revendications 1 à 8, **caractérisé en ce que** le système de mesure (10.1, 10.2) utilisé pour déterminer la pression partielle de $CO_2$ dans le contenu liquide (40) du réacteur à biogaz (100, 200) est relié à une unité d'analyse (20.1, 20.2) qui émet les valeurs de mesure de la pression partielle de $CO_2$ en sortie ou convertit les valeurs mesurées en pression partielle de $CO_2$.

**10.** Procédé selon l'une des revendications 1 à 9, **caractérisé en ce que** la génération de méthane est une méthanisation biologique, en particulier une méthanisation biologique selon un procédé *in situ* dans un procédé de conversion d'électricité en gaz, dans lequel de l'hydrogène (59) et, le cas échéant, du dioxyde de carbone (60) sont ajoutés à les substances organique dans le réacteur anaérobie (200).

**11.** Procédé selon la revendication 10, **caractérisé en ce que** du dioxyde de carbone et/ou du substrat, en particulier un substrat acide ou pauvre en acide, sont ajoutés au contenu liquide du réacteur (40) en fonction des valeurs déterminées de pression partielle de $CO_2$ ($pCO_2$).

**12.** Procédé selon la revendication 11, **caractérisé en ce qu'**une plage optimale des valeurs de pression partielle de $CO_2$ d'un bioréacteur (200) est choisie, en particulier en fonction du volume du réacteur, de la température, de la profondeur d'installation du système de mesure (10.1, 10.2), des substances organique à transformer, de telle sorte que si les valeurs de $pCO_2$ optimales choisies ne sont pas atteintes, l'apport de $CO_2$ (60) et/ou l'apport de substrat au bioréacteur (200) sont augmentés et que, si les valeurs de $pCO_2$ optimales choisies sont dépassées, l'apport de $CO_2$ (60) et/ou l'apport de substrat au bioréacteur (200) sont réduits.

**13.** Procédé selon l'une des revendications 10 à 12, **caractérisé en ce qu'**un autre critère pris en compte pour la stabilité du processus est la sursaturation en $CO_2$ du contenu liquide du réacteur (40), sachant que le pH du contenu du réacteur (40) est mesuré et que l'état de saturation maximale en $CO_2$ $pCO_{2\,HH}$ est calculé en fonction du pH de la manière qui suit :

$$pCO_{2\,HH} = 10^{pKa-pH} \times (p_{air} + p_{esp.gaz} + p_{prof})$$

où

$pCO_{2\,HH}$ représente l'état de saturation maximale en $CO_2$ selon l'équation de Henderson et Hasselbach élargie,
$p_{esp.gaz}$ représente la pression dans l'espace de gaz du réacteur (200) et
$p_{prof}$ représente la correction de la pression par la profondeur de l'emplacement d'installation (48) du système de mesure de la $pCO_2$ (10.2) à l'intérieur du réacteur (200) ;
l'état de saturation en $CO_2$ maximal $pCO_{2\,HH}$ est utilisé avec la valeur de $pCO_2$ mesurée pour calculer la sursaturation en $CO_2$ du contenu liquide du réacteur (40) $pCO_{2\,OS}$ de la manière suivante :

$$pCO_{2\,OS} = pCO_{2\,act} - pCO_{2\,HH}$$

où
$pCO_{2\,OS}$ est la sursaturation en $CO_2$ du contenu liquide du réacteur (40),
$pCO_{2\,HH}$ représente l'état de saturation en $CO_2$ maximal,
$pCO_{2\,act}$ représente la valeur de $pCO_2$ mesurée ;
et les valeurs de pression partielle de $CO_2$ dans le contenu liquide (40) du bioréacteur (200) sont ajustées par l'ajout correspondant de dioxyde de carbone (60) et/ou de substrat de manière à obtenir :

$$1{,}2 \times pCO_{2\,HH} < pCO_{2\,OS} < 3 \times pCO_{2\,HH}.$$

Fig. 1

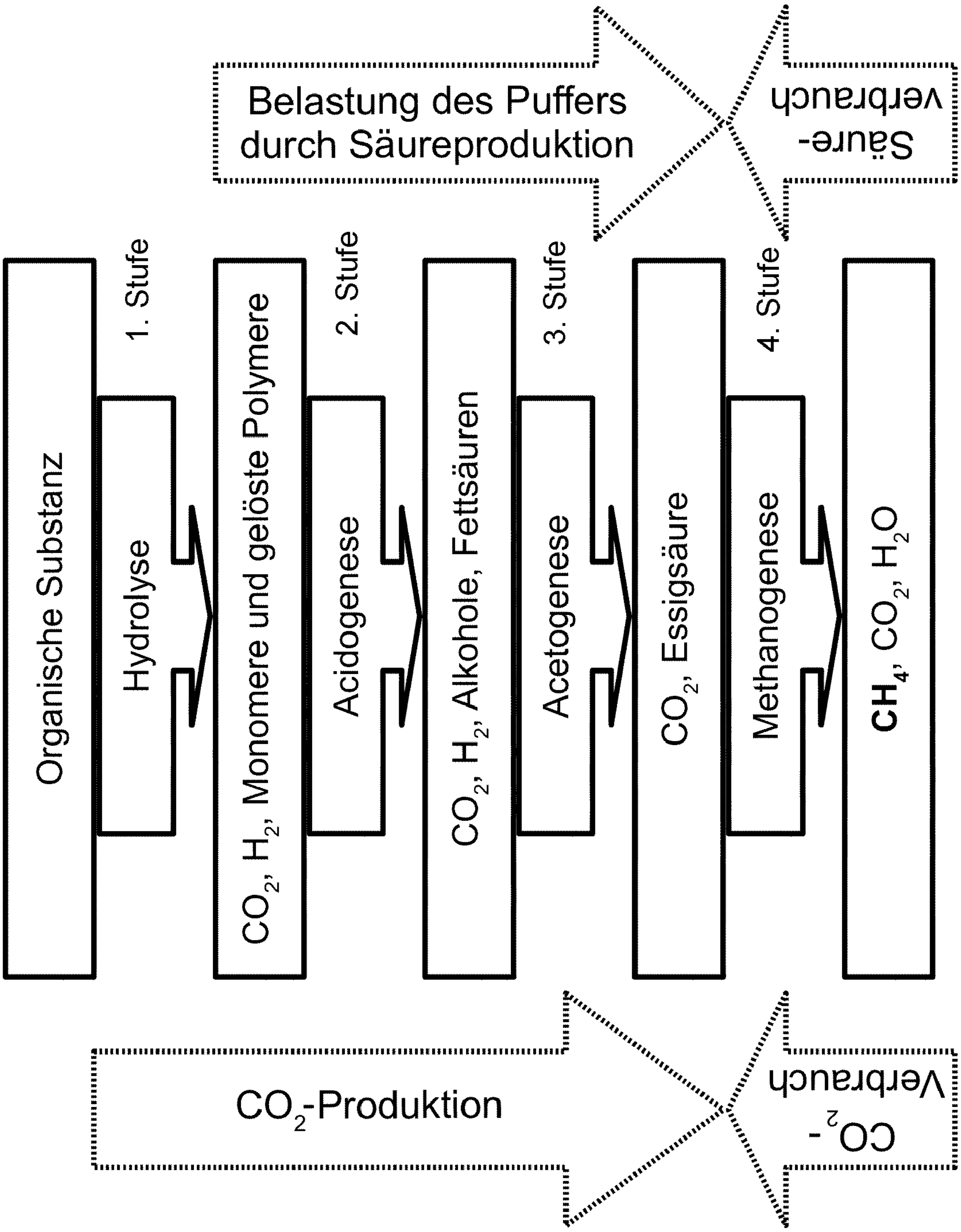
Organische Substanz
1. Stufe
Hydrolyse
$CO_2$, $H_2$, Monomere und gelöste Polymere
2. Stufe
Acidogenese
$CO_2$, $H_2$, Alkohole, Fettsäuren
3. Stufe
Acetogenese
$CO_2$, Essigsäure
4. Stufe
Methanogenese
**$CH_4$**, $CO_2$, $H_2O$
Belastung des Puffers durch Säureproduktion
Säure-verbrauch
$CO_2$-Produktion
$CO_2$-Verbrauch

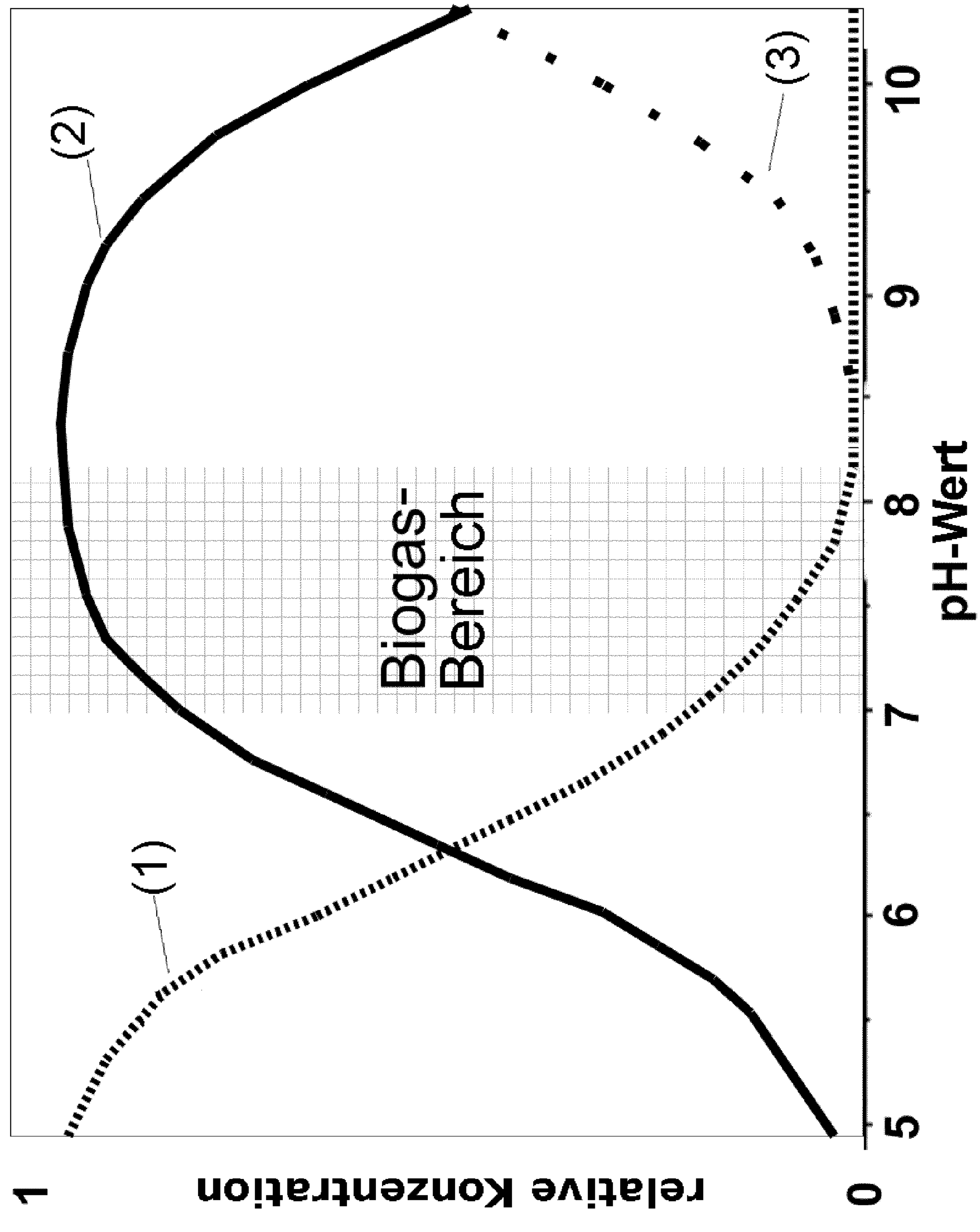
relative Konzentration
1
0
pH-Wert
5
6
7
8
9
10
Biogas-
Bereich
(1)
(2)
(3)

Fig. 2

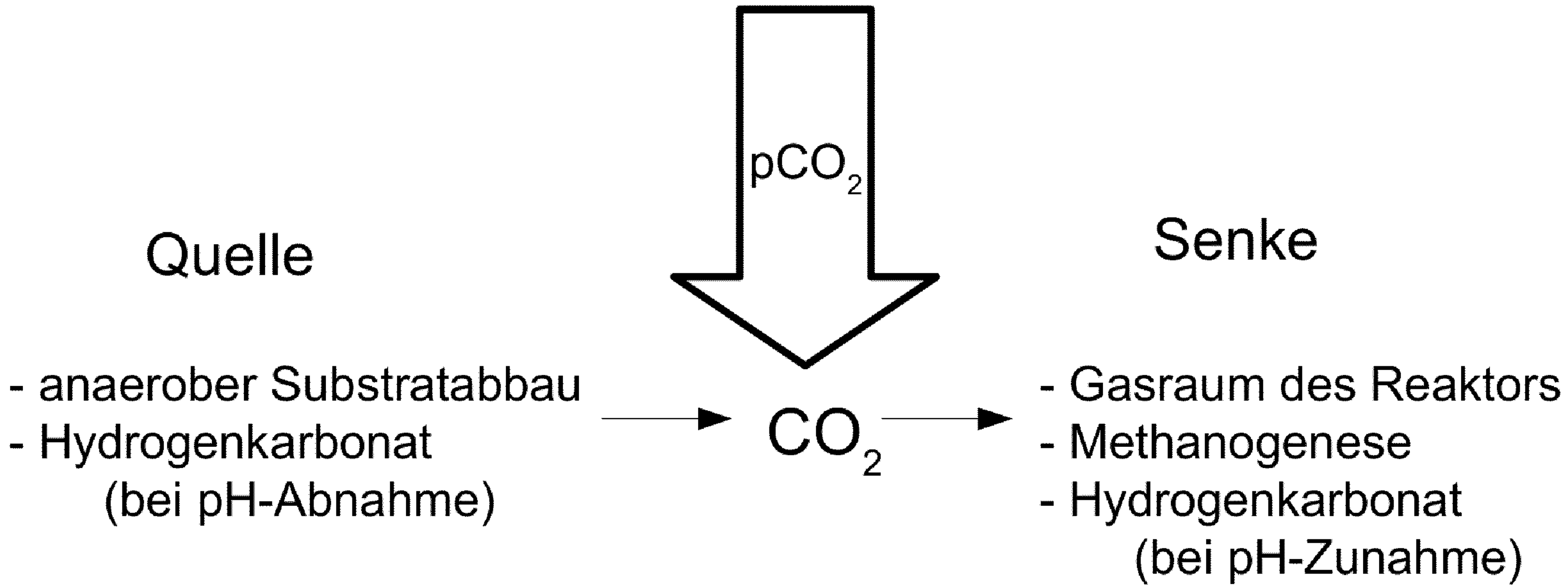
pCO2
Quelle
- anaerober Substratabbau
- Hydrogenkarbonat
(bei pH-Abnahme)
CO2
Senke
- Gasraum des Reaktors
- Methanogenese
- Hydrogenkarbonat
(bei pH-Zunahme)

Fig. 3

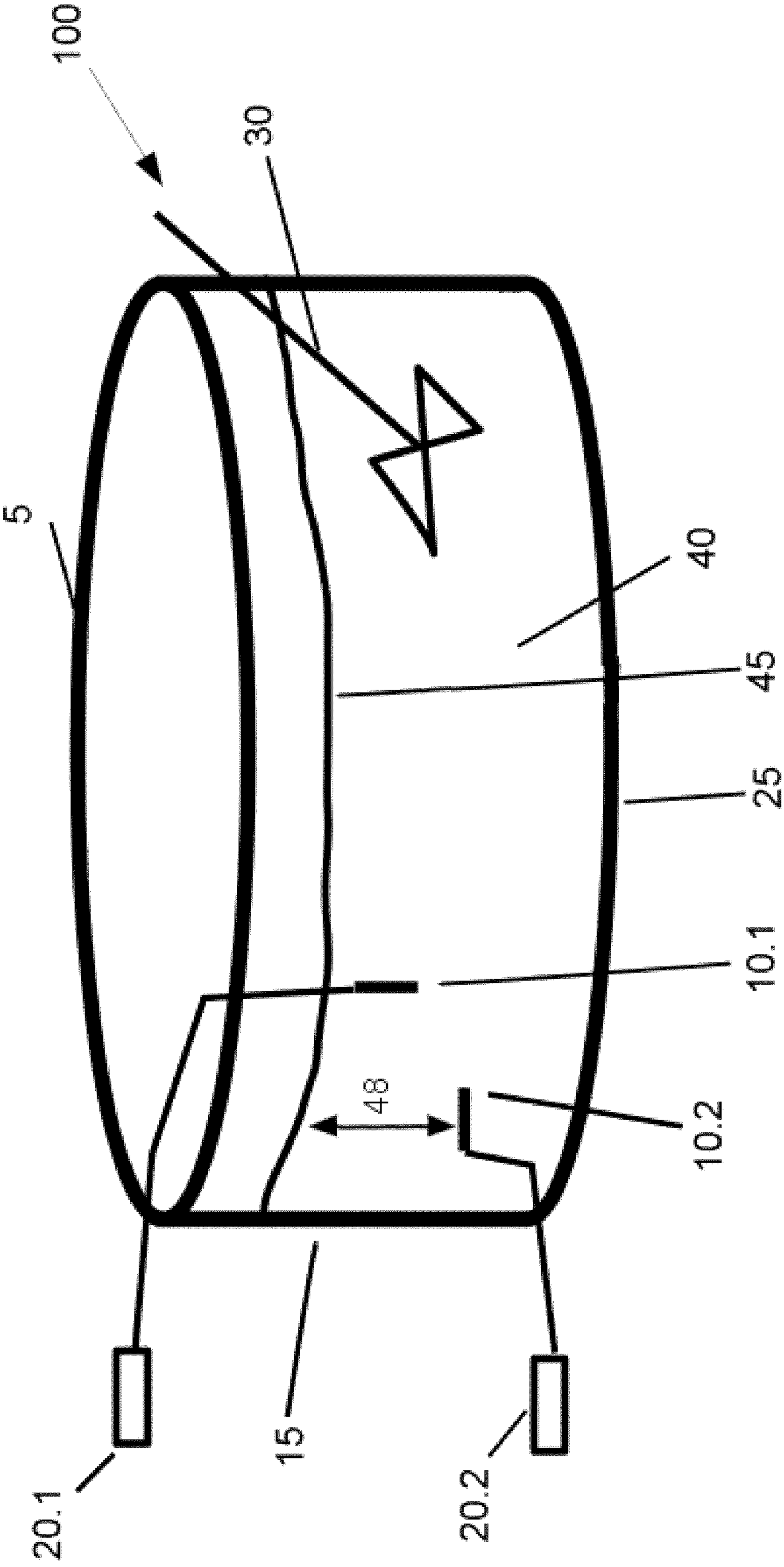


Fig. 4

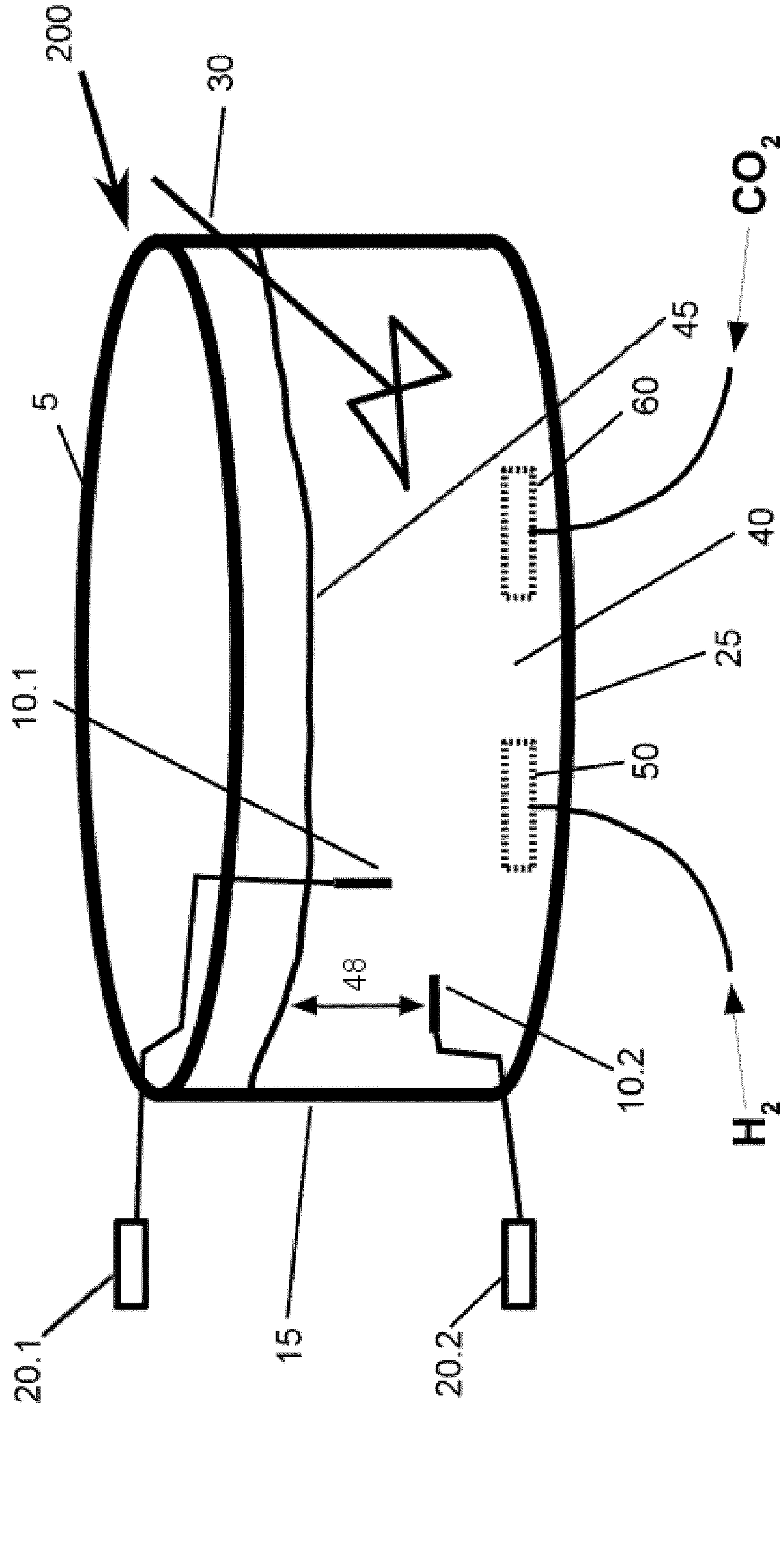


Fig. 5

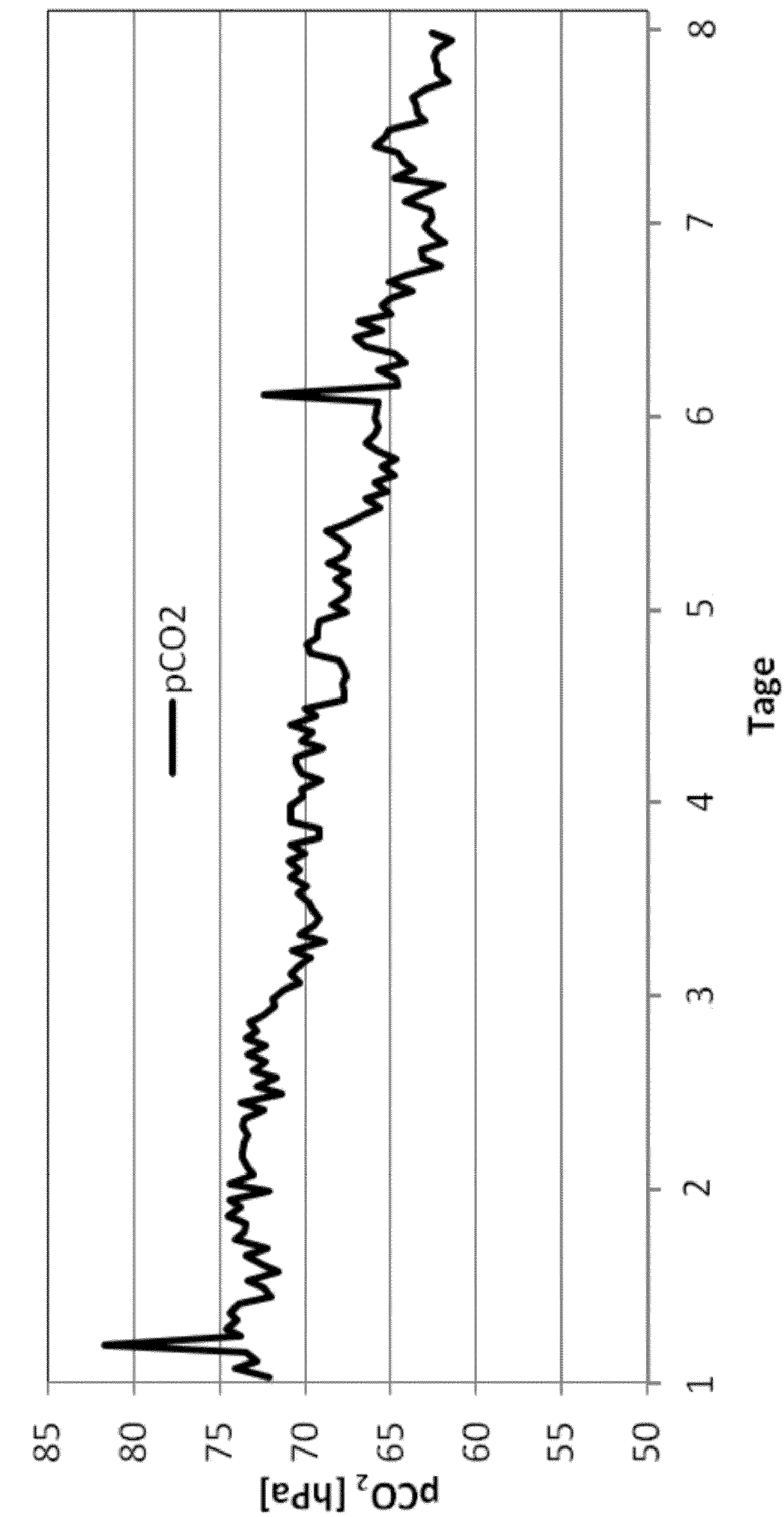


Fig. 6

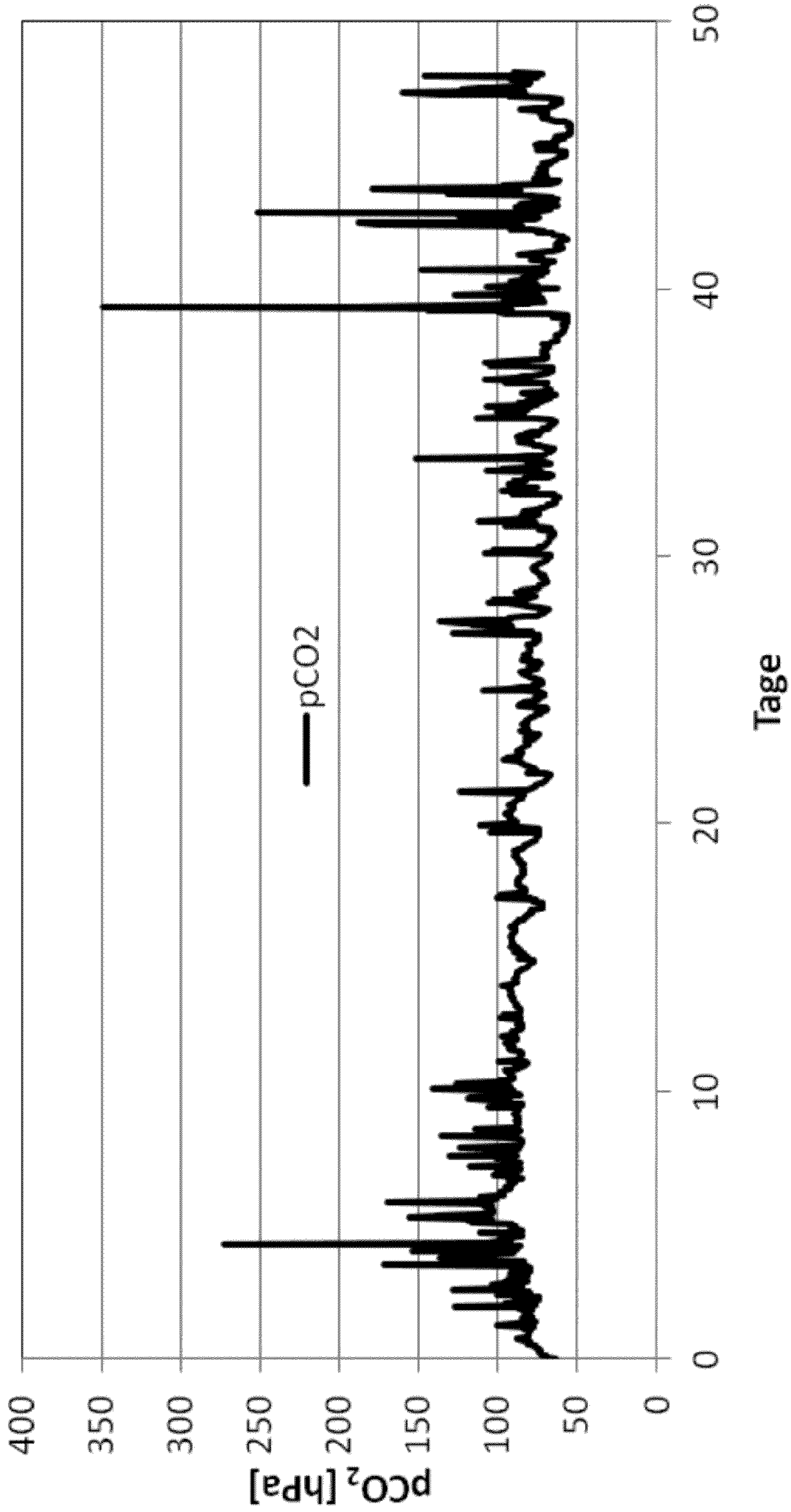
pCO2
Tage
pCO$_2$ [hPa]
0
10
20
30
40
50
0
50
100
150
200
250
300
350
400

**Fig. 7**

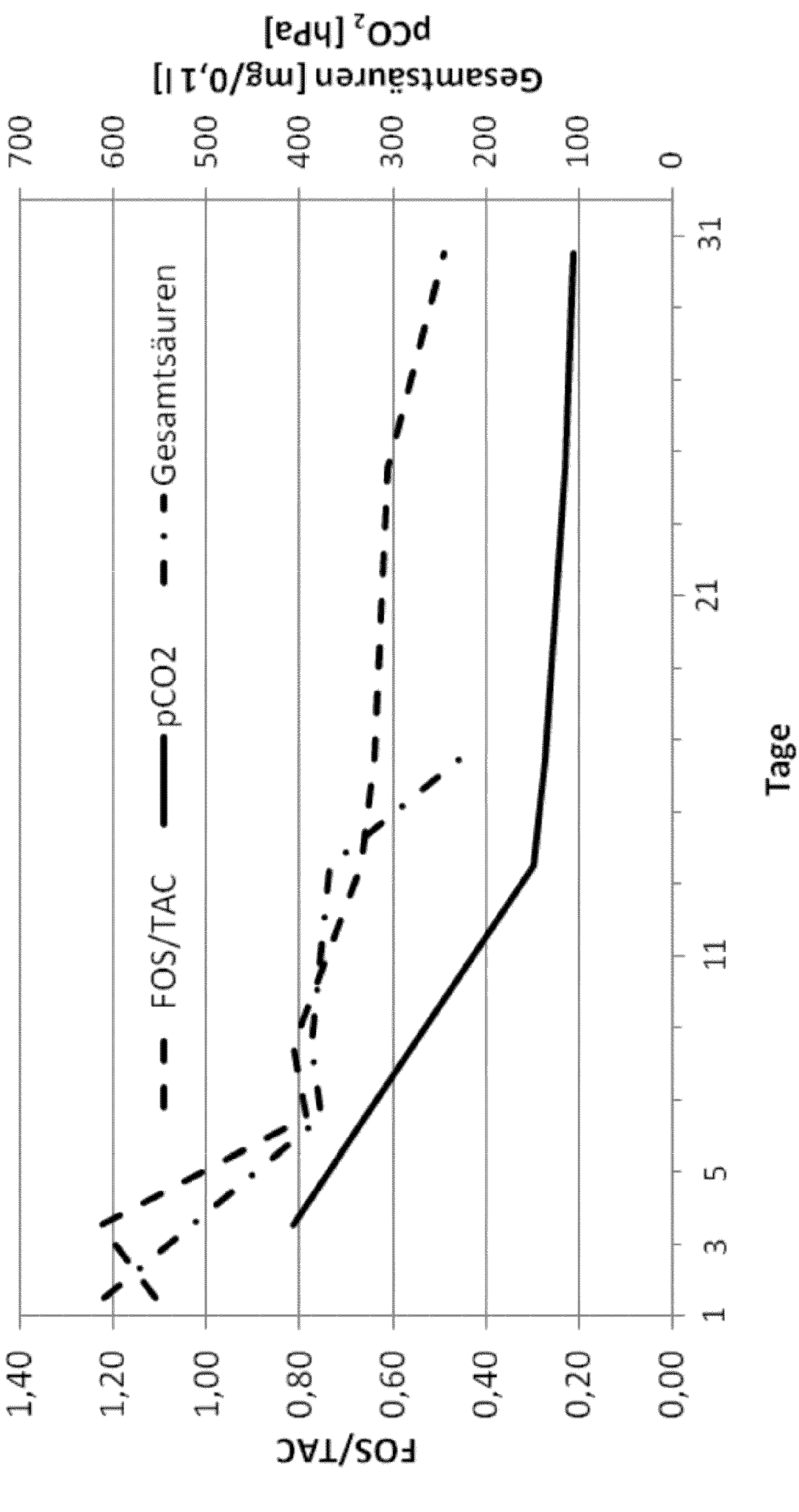
FOS/TAC
1,40
1,20
1,00
0,80
0,60
0,40
0,20
0,00
Gesamtsäuren [mg/0,1 l]
pCO₂ [hPa]
700
600
500
400
300
200
100
0
FOS/TAC
pCO2
Gesamtsäuren
1
3
5
11
21
31
Tage

Fig. 8

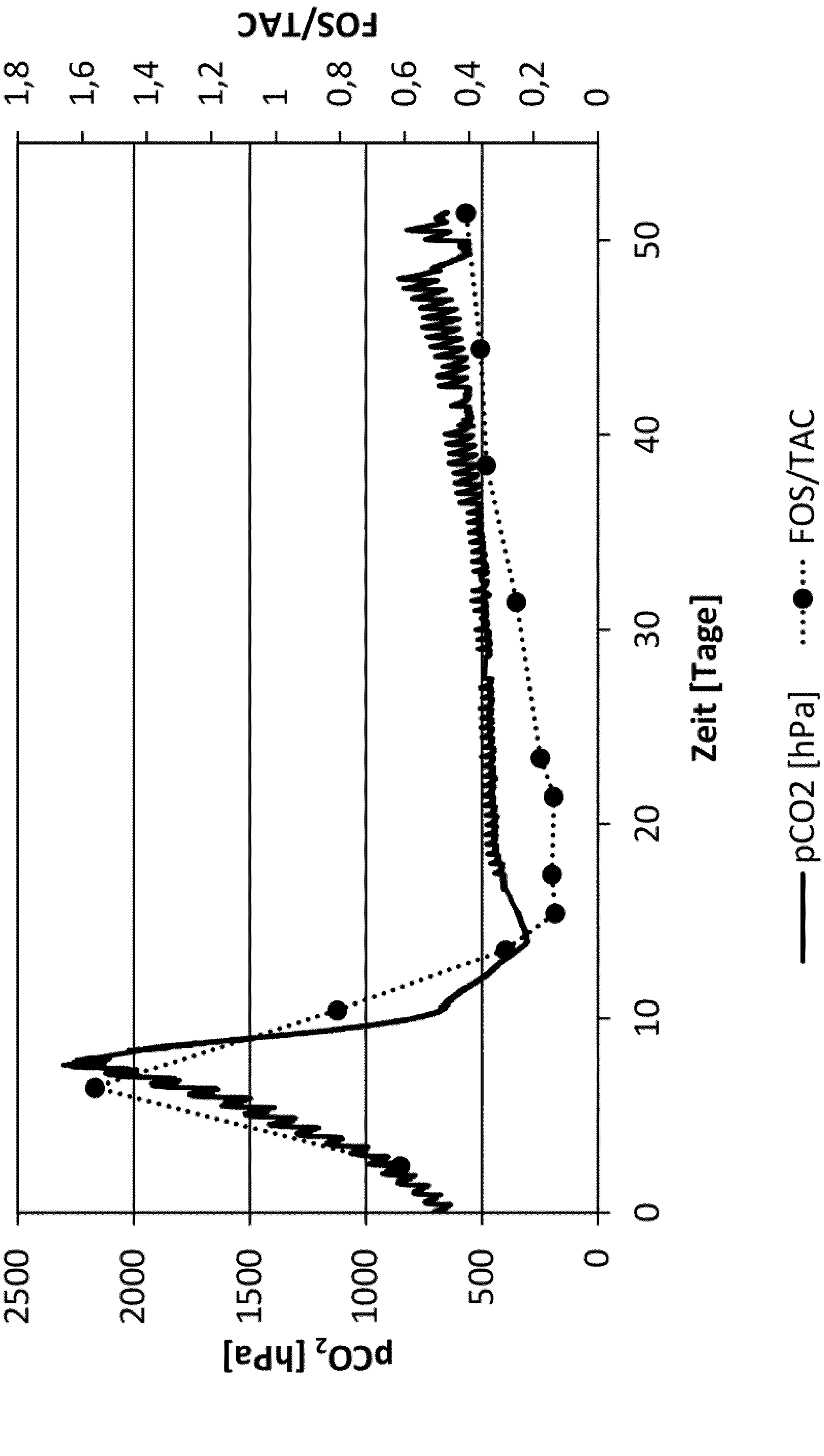
FOS/TAC
1,8
1,6
1,4
1,2
1
0,8
0,6
0,4
0,2
0
2500
2000
1500
1000
500
0
pCO$_2$ [hPa]
0
10
20
30
40
50
Zeit [Tage]
pCO2 [hPa]
FOS/TAC

Fig. 9

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 3427976 A1
- DE 102014006501 A1
- DE 102010043779 A1
- WO 2007014717 A1
- EP 2078947 A2
- DE 102016013068 A1
- WO 2014128300 A1
- DE 102011110638 A1
- DE 102011015415 A1
- DE 102009053593 A1
- DE 102014103311 A1
- WO 2013060331 A1
- DE 102016000070 A1
- WO 2018108810 A1
- US 4444882 A
- WO 2014140703 A1


### In der Beschreibung aufgeführte Nicht-Patentliteratur


- **VOß, E. ; WEICHGREBE, D. ; ROSENWINKEL, K.-H.** FOS/TAC: Herleitung, Methodik, Anwendung und Aussagekraft. *Internationale Wissenschaftstagung Biogas Science,* 2009, vol. 3, 675-682
- **NGUYEN, D ; GADHAMSHETTY, V. ; NITAYAVARDHANA, S. ; KHANAL, S. K.** Automatic process control in anaerobic digestion technology: A critical review. *Bioresource Technology,* 2015, vol. 193, 513-522
- **WEILAND, P.** Biogas production: current state and perspectives. *Applied Microbiology and Biotechnology,* 2010, vol. 85 (4), 849-60
- **BOE, K. ; BATSTONE, D. J. ; STEYER, J.-P. ; ANGELIDAKI, I.** State indicators for monitoring the anaerobic digestion process. *Water Research,* 2010, vol. 44 (20), 5973-80
- **NIELSEN, H. B. ; UELLENDAHL, H. ; AHRING, B. K.** Regulation and optimization of the biogas process: Propionate as a key parameter. *Biomass and Bioenergy,* 2007, vol. 31 (11-12), 820-830
- **STOCKL, A. ; OECHSNER, H.** Near-infrared spectroscopic online monitoring of process stability in biogas plants. *Engineering in Life Sciences,* 2012, vol. 12 (3), 295-305
- **GÖTZ, M ; LEFEBVRE, J. ; MÖRS, F. ; KOCH, A.M. ; GRAF, F. ; BAJOHR, S. ; REIMERT, R. ; KOLB, T.** Renewable Power-to-Gas: A technological and economic review. *Renewable Energy,* 2016, vol. 85, 1371-1390
- **AGNEESSENS, L.M. ; OTTOSEN, L.D.M. ; OTTOSEN, N.V. ; NIELSEN, J.L. ; JONGE, N. ; FISCHER, CH. H. ; KOFOED, M.V.W.** In-situ biogas upgrading with pulse H2 additions: The relevance of methanogen adaption and inorganic carbon level. *Bioresource Technology,* 2017, vol. 233, 256-263
- **LOWER, S. K.** Carbonate equilibria in natural waters. *A Chem1 Reference Text, http://citeseerx.ist.psu.edu/viewdoc/summary?doi=10.1.1.214.2947*
- **STUMM, W ; MORGAN J.J.** Aquatic Chemistry: Chemical Equilibria and Rates in Natural Waters. Wiley, 2012, 1040
- Dissolved Carbon Dioxide
- **LIBES, S.** Introduction to Marine Biogeochemistry. Elsevier, 2009, 928
- **FISCHLIN, A ; BUCHTER, B ; MATILE, L. ; HOFER, P ; TAVERNA, R.** CO2-Senken und -Quellen in der Waldwirtschaft - Anrechnung im Rahmen des Kyoto-Protokolls. *Umwelt-Wissen Nr. 0602, Bundesamt für Umwelt, Bern,* 2006, vol. 45, 1-47
- **TIAN-GEN CHANG ; XIN-GUANG ZHU.** Source-sink interaction: a century old concept under the light of modern molecular systems biology. *Journal of Experimental Botany,* 2017, vol. 68 (16), 4417-4431
- **MILLERO, F.J.** Thermodynamics of the carbon dioxide system in the oceans. *Geochim. Cosmochim. Acta,* 1995, vol. 59, 661-677
- **WEISS, R.F.** Carbon dioxide in water and seawater: the solubility of a non-ideal gas. *Mar. Chem,* 1974, vol. 2, 203-215